# EUROPEAN PATENT SPECIFICATION

(11) **EP 4 537 772 B1**
(45) Date of publication and mention of the grant of the patent: **22.07.2026**
(21) Application number: 24205474.0
(22) Date of filing: 09.10.2024
(51) Int. Cl.: A61B 17/17, A61B 17/74

(54) **INTRAMEDULLARY NAIL AIMING SYSTEMS**
MARKNAGELZIELSYSTEME
SYSTÈMES DE VISÉE DE CLOU INTRAMÉDULLAIRE

(30) Priority: 09.10.2023 US 202318483194
(43) Date of publication of application: 16.04.2025
(73) Proprietor: Globus Medical, Inc, Audubon, PA 19403 (US)
(72) Inventor: MYERS, Daniel J., Conshohocken, 19428 (US); YUCHIMIUK, Nathaniel, Sanatoga, 19464 (US); PHILIP, Jonan A., Phoenixville, 19460 (US); GRAY, Jason, East Greenville, 18041 (US); VENT, Justin, Havertown, 19083 (US); PETERSON, John, Conshohocken, 19428 (US)
(74) Representative: Morabito, Sara

(56) References cited:
- AT-A1- 525 310
- US-A1- 2014 214 045
- US-A1- 2020 113 609

## Description

### FIELD OF THE INVENTION

The present disclosure relates to surgical devices, and more particularly, to intramedullary nail implantation for treatment of bone fractures, for example, for trauma applications.

### BACKGROUND OF THE INVENTION

Bone fractures of long bones, such as the femur, tibia, and humerus, may be treated by fixation of the bone to stabilize and align the fractured bone, facilitating the healing process. In some instances, when a bone is fractured, an intramedullary nail may be introduced through the medullary cavity of the bone to immobilize the fracture fragments and stabilize the long bone. In the case of the femur, trochanteric nails may be inserted into the canal of the femur through the greater trochanter to stabilize a variety of proximal femur fractures. The trochanteric nails may be secured with one or more fixation screws, for example, for proximal and/or distal fixation. Femoral neck fixation may include insertion of a fixation screw into the femoral neck/head at an angle relative to the intramedullary nail. One or more aiming or targeting instruments may be used to facilitate insertion of the intramedullary nail and the respective fixation screws. There remains a need, however, for improved intramedullary systems and instruments for aligning the components to provide stabilization to the bone and improved patient outcomes.

US2014/214045 and AT 525 310 A1 describe each a targeting system known in the art.

### SUMMARY OF THE INVENTION

Intramedullary nail aiming systems, instruments, and method of treatment are provided. Intramedullary nails may be suitable for implanting within a medullary canal of a fractured long bone, such as the femur, and subsequently providing proximal fixation and/or distal fixation, for example, with one or more anchors, fasteners, fixation screws, or the like. The aiming systems and instruments may be configured to ensure optimal alignment and placement of one or more components of the intramedullary nail system.

According to the invention it is provided a targeting system having the features of claim 1. Further advantageous aspects of the invention are set forth in the dependent claims.

According to one embodiment, a targeting system for inserting a lag screw into an intramedullary nail is provided. The proximal targeting system includes an intramedullary nail and a nail insertion handle. The intramedullary nail has a proximal end with a proximal through opening and a distal end. The nail insertion handle includes a coupling portion to removably couple to the proximal end of the intramedullary nail, a handle portion, and an aiming guide having an opening that aligns with the proximal through opening in the intramedullary nail. The handle portion includes an anteversion alignment system configured to optimally insert a lag screw into the proximal through opening in the intramedullary nail, an anti-rotation system configured to stabilize the bone when the lag screw is inserted, and a lag screw aiming assembly configured to reliably insert down to the bone, thereby defining a trajectory to the proximal opening in the intramedullary nail.

The proximal targeting system may include one or more of the following features. The handle portion may define an anteversion guide wire hole along a center plane of the nail insertion handle and a pair of radiopaque goal posts positioned on opposite sides of the guide wire hole. The radiopaque goal posts may include two parallel rows of pins retained in the handle portion, and when the goal posts are centered in parallel and a guide wire positioned through the anteversion guide wire hole is centered with a neck and head of the bone, an optimal anteversion position is found for lag screw insertion. The anti-rotation system may include a plurality of anti-rotation holes, anti-rotation sleeves positionable through the holes, and anti-rotation guide wires positionable through the sleeves. The anti-rotation holes may each include a retention pin configured to secure the anti-rotation sleeve in the anti-rotation hole. The anti-rotation holes may include an upper pair of holes converging distally at a first angle and a lower pair of hole converging distally at a second angle such that the second angle is greater than the first angle. The lag screw aiming assembly may include a driver sleeve positionable through the opening in the aiming guide, a wire sleeve with distal cutting flutes positionable through the driver sleeve, and a trocar positionable through the wire sleeve.

According to one embodiment, a targeting system may include an intramedullary nail, a nail insertion handle, a driver sleeve, a wire sleeve, and a guide wire. The intramedullary nail has a proximal end and a distal end. The nail insertion handle includes a coupling portion to removably couple to the proximal end of the intramedullary nail, a handle portion, and an aiming guide having an opening that aligns with an opening in the intramedullary nail. The driver sleeve is positionable through the opening in the aiming guide. The wire sleeve is positionable through the driver sleeve, and the wire sleeve has a plurality of graduated markings. The guide wire is positionable through the wire sleeve. When the wire sleeve is flush with the guide wire, a reading of the driver sleeve next to the graduated markings indicates an optimal lag screw length.

The targeting system may include one or more of the following features. The driver sleeve may include a tubular body with a plurality of teeth configured to engage a ratchet within the aiming arm. A distal tip of the wire sleeve may include cutting flutes. The wire sleeve may include an enlarged head portion defining a spring cut that enables the wire sleeve to snap into the driver sleeve. The targeting system may also include a trocar positionable through the wire sleeve. The trocar may include a shaft with a sharp distal tip configured to facilitate insertion through soft tissue. The trocar may include a head portion with a male mating member receivable in a corresponding female mating member in the wire sleeve such that the trocar and wire sleeve turn together to help drive through soft tissue. The trocar may have a bent shaft to facilitate retention within the wire sleeve.

It is further described but does not form part of the invention a method of inserting an intramedullary nail in a bone of a patient may include one or more of the following steps in any suitable order: (1) inserting a coring reamer instrument into an axial end of a long bone to access a medullary canal, the coring reamer having a hollow coring tip with circumferential teeth configured to cut and collect bone; (2) securing an intramedullary nail to a nail insertion handle with a keyed interface and a connection bolt, the nail insertion handle includes an implant holder including a coupling portion and a handle portion, wherein the coupling portion includes keyed tabs configured to mate with corresponding recesses in the proximal end of the intramedullary nail, and the connection bolt passes through internal threads in the implant holder and threads into the intramedullary nail; and (3) inserting the intramedullary nail into the medullary canal with the nail insertion handle. In one embodiment, the nail insertion handle includes an anteversion guide wire hole located along a center plane of the handle and a pair of radiopaque goal posts positioned on either side of the guide wire hole. The method may further include (4) positioning a guide wire through the guide wire hole and adjusting the nail insertion handle until the goal posts are parallel and centered with the intramedullary nail and the guide wire is centered through a neck and head of the bone, thereby ensuring an optimal anteversion position for lag screw insertion. The method may include (5) attaching an aiming arm to the handle portion of the nail insertion handle with a pair of alignment pins and an attachment knob. In one embodiment, the aiming arm includes a plurality of anti-rotation sleeve holes. The method may include (6) positioning anti-rotation sleeves through the sleeve holes and positioning anti-rotation guide wires through the anti-rotation sleeves, wherein each anti-rotation sleeve hole includes a retention pin configured to secure the anti-rotation sleeve in the anti-rotation hole. The method may include (7) inserting a driver sleeve through an opening in the aiming arm, inserting a wire sleeve through the driver sleeve, and inserting a trocar through the wire sleeve; and/or (8) attaching a driver to a lag screw and inserting the lag screw through the driver sleeve and into an opening in the intramedullary nail.

According to one embodiment, a targeting system for inserting a distal fastener into an intramedullary nail includes an intramedullary nail, a nail insertion handle, a distal aiming arm, a deflection assembly, a targeting module. The intramedullary nail has a proximal end and a distal end with a distal through opening. The nail insertion handle includes a coupling portion to removably couple to the proximal end of the intramedullary nail and a handle portion. The distal aiming arm is attachable to the handle portion of the nail insertion handle, wherein the distal aiming arm includes a plurality of dovetail recesses. The deflection assembly includes a dovetail assembly for attachment to the dovetail recesses and an adjustment assembly for adjusting vertical position of the deflection assembly relative to the distal aiming arm. The targeting module is attachable to the deflection assembly and defines an opening therethrough. The sleeve is positionable through the opening in the targeting module. The deflection assembly is adjustable to account for deflection of the intramedullary nail, thereby defining a trajectory to the distal through opening in the intramedullary nail.

The distal targeting system may include one or more of the following features. The distal aiming arm may include an attachment head with two mirrored connection surfaces. The two mirrored connection surfaces may have two angles that act in different planes. The distal aiming arm may include a plate with an arcuate bend and the dovetail recesses are defined into inner and outer faces of the plate to accommodate incremental placement of the deflection assembly. Each dovetail recess may define a ball detent divot configured to receive a corresponding ball detent on the deflection assembly. The adjustment assembly may include a fine adjustment knob and a coarse adjustment button for vertically adjusting the deflection assembly. The targeting module may include a plurality of overlapping holes. The targeting module may be attached to the deflection assembly with a tenon configured to fit within a corresponding groove in a bottom surface of the deflection assembly. The sleeve may have a D-shaped shaft with a flat that interfaces with a retention pin in the targeting module.

According to one embodiment, a targeting system includes a distal aiming arm including a plurality of dovetail recesses, a deflection assembly including a dovetail assembly for attachment to the dovetail recesses and an adjustment assembly for adjusting a vertical position of the deflection assembly relative to the distal aiming arm, a targeting module attachable to the deflection assembly, the targeting module defining an opening therethrough, and an alignment display with markings to determine an optimal targeting position.

The distal targeting system may include one or more of the following features. The markings on the alignment display may include radiopaque measurement markings viewable on x-ray. The dovetail assembly may include a moveable block and a cam lock attached to a foot having a ball detent. The foot may include a male dovetail projection with a planar outer surface and an arched bottom surface configured to be received into one of the dovetail recesses in the distal aiming arm. The cam lock may include a cam lever connected to the foot and an internal compression spring, which maintains preload on the foot. The deflection assembly may include an outer casing with a handle portion for retaining the dovetail assembly, a base for securing the targeting module, and a neck for retaining the adjustment assembly. The adjustment assembly may include a fine adjustment knob with a rotatable knob attached to a threaded shaft, which is threadedly engaged with the outer casing. The adjustment assembly may include a coarse adjustment button with a threaded through opening that is retained in the neck of the casing. The threaded shaft extends through the coarse adjustment button, and in one position, the threaded shaft is engaged with the coarse adjustment button and in another position, the threaded shaft is disengaged from the coarse adjustment button.

It is further described a method of aligning fasteners to an intramedullary nail may include one or more of the following steps in any suitable order: (1) inserting an intramedullary nail with a distal through opening into a medullary canal of a patient with a nail insertion handle; (2) attaching a distal aiming arm to the nail insertion handle, the distal aiming arm having a plurality of dovetail recesses; (3) attaching a deflection assembly to the dovetail recesses; (4) attaching a targeting module to the deflection assembly, the targeting module defining an opening therethrough; (5) positioning a sleeve through the opening in the targeting module; and (6) adjusting a vertical position of the deflection assembly relative to the distal aiming arm to account for deflection of the intramedullary nail and defining a trajectory to the distal through opening in the intramedullary nail. The method may also include (7) viewing an alignment display on the targeting module to adjust the vertical position of the targeting module; and/or (8) attaching a driver to a locking screw and inserting the locking screw through the sleeve and into the distal through opening in the intramedullary nail.

Also provided are kits including intramedullary nails of varying shapes and sizes, bone anchors, fasteners, insertion tools, proximal aiming systems, distal aiming systems, K-wires, and other tools and devices which may be suitable for aiding in the surgical procedure.

### BRIEF DESCRIPTION OF THE DRAWINGS

A more complete understanding of the present invention, and the attendant advantages and features thereof, will be more readily understood by reference to the following detailed description when considered in conjunction with the accompanying drawings, wherein:
FIG. 1 shows a proximal femur with an anteversion alignment system coupled to a trochanteric nail and a lag screw optimally inserted into the femoral neck and head via the anteversion alignment system according to one embodiment;
FIG. 2 shows the anteversion alignment system attached to the trochanteric nail configured for aligning the trochanteric nail to the optimal anteversion angle prior to lag screw insertion;
FIG. 3 is a lateral close-up view of the anteversion alignment system including radiopaque goal posts and the anteversion K-wire according to one embodiment;
FIGS. 4A-4C show a workflow including misaligned and aligned configurations for the anteversion alignment system according to one embodiment;
FIG. 5 shows the anteversion alignment system with placement of the lag screw at the optimal anteversion location;
FIG. 6 shows an anti-rotation system integrated into a trochanteric nail aiming arm according to one embodiment, which is configured to stabilize the femoral neck and head when the lag screw is inserted;
FIG. 7 shows an anteroposterior view of the anti-rotation system with converging pathways for the anti-rotation guide wires according to one embodiment;
FIGS. 8A-8D show examples of anti-rotation holes with a retention pin configured for locking an anti-rotation sleeve therein;
FIG. 9 shows a lag screw aiming assembly according to one embodiment, which is configured to reliably insert a sleeve assembly down to bone during a trochanteric nailing procedure;
FIG. 10A is one embodiment of a lag screw sleeve assembly including a lag screw driver sleeve, a lag screw wire sleeve, and a lag screw trocar;
FIG. 10B is another embodiment of a lag screw sleeve assembly including a lag screw driver sleeve, a lag screw wire sleeve, and a lag screw Trocar;
FIGS. 11A-11C show a ratchet mechanism configured to lock the lag screw driver sleeve to the aiming arm according to one embodiment;
FIGS. 12A-12D show the lag screw assembly components including the connection between the lag screw wire sleeve and the lag screw driver sleeve and the interface between the trocar and the lag screw wire sleeve;
FIG. 13 shows the lag screw aiming assembly with a quick connect handle according to one embodiment;
FIGS. 14A-14B show the quick connect handle coupled to the trocar according to one embodiment;
FIG. 15 shows lag screw guide wire insertion through the lag screw sleeve assembly according to one embodiment;
FIG. 16 shows an embodiment for measuring the length of the lag screw guide wire with the lag screw wire sleeve;
FIGS. 17A-17B are isometric views of a push-connect handle assembly with different handle types;
FIGS. 18A-18B are sectional views of the push-connect handle assembly according to one embodiment;
FIGS. 19A-19B are sectional views of the female push-connect assembly and the male and female connection when assembled;
FIG. 20 shows an embodiment of a trochanteric nail proximal aiming system according to one embodiment;
FIGS. 21A-21D show the insertion handle assembly and nail connection geometry according to one embodiment;
FIGS. 22A-22B show examples of nail connection geometry for different nail types;
FIGS. 23A-23C show the connection bolt for securing the insertion handle to the trochanteric nail according to one embodiment;
FIGS. 24A-24B show assembly of the insertion handle and the aiming arm according to one embodiment;
FIGS. 25A-25C show assembly of the sub-components of the aiming arm according to one embodiment;
FIGS. 26A-26B show the proximal aiming system with an anti-rotation wire sleeve according to one embodiment;
FIGS. 27A-27C show the lag screw driver sleeve with ratchet teeth according to one embodiment;
FIGS. 28A-28B show the lag screw wire sleeve with lag screw length calibrations according to one embodiment;
FIG. 29 is a lag screw trocar with a bent shaft according to one embodiment;
FIGS. 30A-30B show assembly of the lag screw sleeve with the aiming arm according to one embodiment;
FIGS. 31A-31C show the lag screw driver and lag screw retention rod according to one embodiment;
FIGS. 32A-32B show the proximal aiming system with the lag screw driver and retention rod used for targeted lag screw insertion according to one embodiment;
FIGS. 33A-33D show an impaction mallet used to insert and remove implants according to one embodiment;
FIG. 34 shows the impaction mallet with a slap removal shaft according to one embodiment;

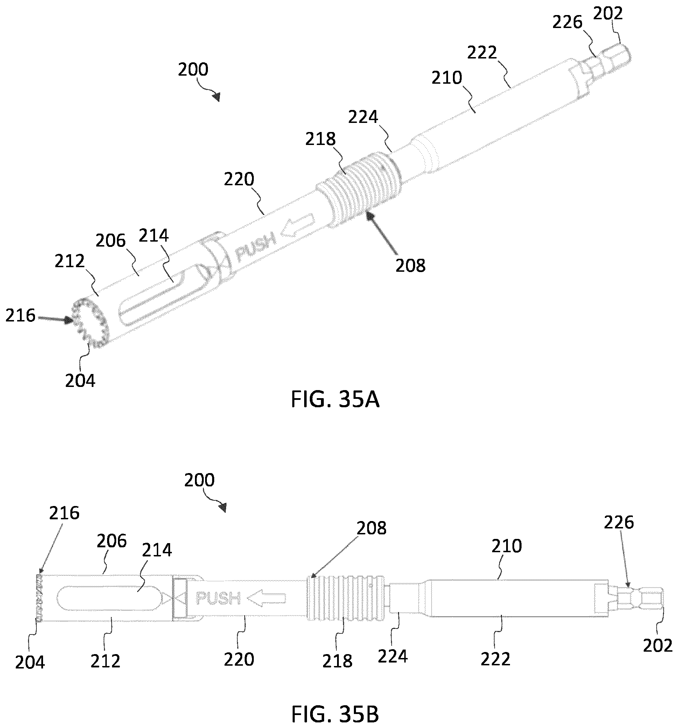

FIGS. 35A-35B show perspective and side views, respectively, of a coring reamer having a coring tip, a core ejector, and a reamer shaft according to one embodiment, which is configured to create a hole in the axial end of a long bone to access the medullary canal;
FIGS. 36A-36B show cross-sectional views of the engagement between the core ejector and reamer shaft according to one embodiment;
FIG. 37 is one embodiment of a guidewire pusher to keep the ball-tip guidewire in place as the reamer and reamer shaft are removed from the medullary canal;
FIGS. 38A-38B shows examples of ball-tip wire engagement with the guidewire pusher;
FIGS. 39A-39B show additional examples of ball-tip wire engagement with the guidewire pusher;
FIG. 40 shows an exploded view of a nail assembly including an insertion handle, nail connection bolt, and trochanteric nail according to one embodiment;
FIGS. 41A-41B shows the nail connection bolt and a mating interface on the guidewire pusher according to one embodiment;
FIG. 42 shows an assembly for using the guidewire pusher through the trochanteric nail according to one embodiment;
FIG. 43 shows a close-up view of the guidewire pusher inserted through the nail insertion handle and trochanteric nail according to one embodiment;
FIGS. 44A-44B show a distal aiming arm attachable to different nail insertion handles, which are coupled to different types of intramedullary nails according to one embodiment;
FIGS. 45A-45B show a distal aiming assembly including the distal aiming arm attached to the nail insertion handle, a deflection assembly with a targeting module, and a sleeve for targeting distal holes of the intramedullary nail according to one embodiment;
FIGS. 46A-46E show the distal aiming arm including an attachment head and attachment dovetails along the plate according to one embodiment;
FIGS. 47A-47G show the deflection assembly that attaches to the distal aiming arm according to one embodiment;
FIGS. 48A-48C show examples of targeting modules, which are attachable to the deflection assembly;
FIG. 49 shows the targeting assembly with the targeting module attached to the deflection assembly according to one embodiment;
FIGS. 50A-50C show examples of anti-rotation holes in the targeting module with retention pins configured for locking an anti-rotation sleeve therein; and
FIG. 51 shows an alignment display with markings to indicate the optimal distal targeting according to one embodiment.

### DETAILED DESCRIPTION OF THE INVENTION

Intramedullary nail aiming systems, instruments, and method of treatment are provided. The intramedullary nails may be suitable for implantation within the medullary canal of a fractured long bone with proximal fixation and/or distal fixation, for example, with one or more anchors, fasteners, fixation screws, or the like. In one embodiment, trochanteric nails are inserted into the canal of the femur through the greater trochanter to stabilize a variety of proximal femur fractures. Although generally described with reference to hip fractures of the femur, it will be appreciated that the intramedullary nail systems may be adapted to be used for the fixation of other areas or other long bones as well, such as the tibia, humerus, clavicle, fibula, ulna, radius, bones of the foot, bones of the hand, or other suitable bone or bones.

The targeting or aiming systems may include one or more instruments or systems configured to ensure optimal alignment and placement of one or more components of the intramedullary nail system. In one embodiment, the intramedullary nail aiming system may include an anteversion alignment system configured for aligning a trochanteric nail to the optimal anteversion location prior to lag screw insertion. In another embodiment, the aiming system may include an anti-rotation system configured to stabilize the fracture to prevent rotation when the lag screw is inserted. In another embodiment, the aiming system may include a lag screw aiming assembly configured to enable surgeons to reliably insert a lag screw sleeve down to bone during the trochanteric nailing procedure. In yet another embodiment, the system may include a proximal aiming system and/or a distal aiming system used for aiming and inserting the proximal and distal bone anchors or fasteners into the nail. The instruments may further include reaming instruments, wire pushers, quick connect handles, impaction mallets, and other tools and instruments which may be suitable to enhance or optimize the surgical procedure.

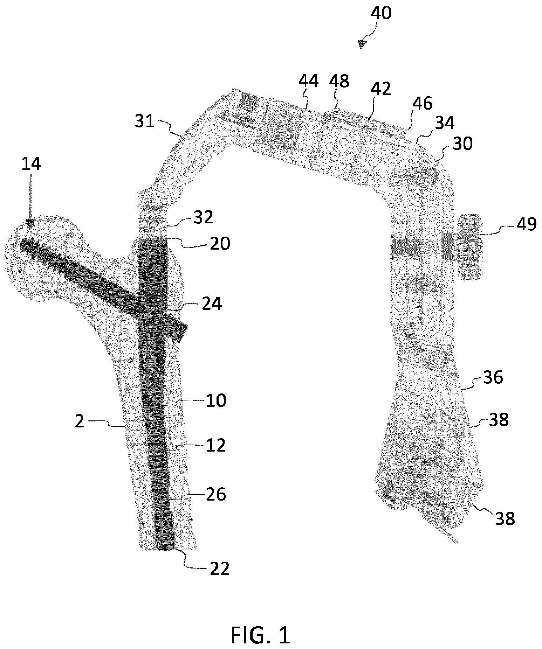

Turning now to the drawing, where like reference numerals refer to like elements, FIG. 1 shows an anteroposterior (A/P) view of a femur bone 2 with an intramedullary nail system 10 according to one embodiment. The intramedullary nail system 10 may include an intramedullary nail 12 and one or more bone anchors 14, fasteners, fixation screws, or the like configured to provide proximal and/or distal fixation. In the case of femur 2, the intramedullary nail 12 may be a trochanteric nail configured to be inserted through the greater trochanter and into the canal of the femur 2 and a proximal anchor 14 may be a lag screw inserted through the femoral neck and head of the femur 2.

Intramedullary nail 12, such as a trochanteric nail, may be a long rod or stem that extends from a first end or proximal end 20 to a second end or distal end 22. The nail 12 is configured to extend longitudinally within the medullary canal of the femur bone 2. The nail 12 may be configured as a cylindrical shaft or tubular rod or the shaft may be configured with any geometrical cross-dimensional shape (e.g., rectangular, oval, elliptical, oblong, polygonal, or the like) that suits the medullary canal. The nail 12 may be substantially straight or bent to mimic the natural anatomical curvature of the long bone. In this embodiment, the intramedullary nail 12 may be configured to be positioned in the proximal end of the femur through the greater trochanter for cephalomedullary fixation. It is envisioned, however, that the intramedullary nail 12 may be configured to be positioned through other approaches and locations (e.g., distal end) depending on the bone (e.g., femur, tibia) and type of fracture.

The proximal end 20 of nail 12 may include one or more proximal openings 24 configured to receive one or more bone anchors 14, fasteners, or proximal fixation devices, such as a threaded lag screw. The lag screw 14 may be configured to extend transversely through the intramedullary nail 12 to secure the proximal end 20 of the nail 12 within the canal. The lag screw 14 may be a calcar screw or other suitable anchor configured to be aimed at the neck and head of the proximal femur. The proximal end 20 of nail 12 may optionally include additional openings 24, for example, for one or more cross-locking devices.

The distal end 22 of the nail 12 may include one or more distal openings 26 configured to receive one or more bone anchors 14, fasteners, or distal fixation devices (not shown). The distal fixation devices may be configured to extend transversely through the distal end 22 of the intramedullary nail 12 to secure the distal end 22 of the nail 12 within the canal. Any of the openings 24, 26 may be threaded or textured (e.g., to receive locking fasteners), non-threaded/non-textured (e.g., to receive compression fasteners), or may be otherwise configured to receive the bone anchors 14.

The intramedullary nails 12 and bone anchors 14 may be available in a variety of lengths, widths, and styles based on the anatomy of the patient. The nails 12 may be configured in both left and right designs, in a mirrored configuration, in order to address the anatomy of both the left and right sides of the patient. The systems may be adapted to secure small or large bone fragments, single or multiple bone fragments, or otherwise secure one or more fractures. In particular, the systems may include a series of intramedullary nails 12 and anchors 14 designed for the fixation of fractures and fragments in diaphyseal, metaphyseal, and/or epiphyseal bone. The nail 12 may come in various shapes, sizes, and designs tailored for specific bones, locations, and types of fractures. Examples of suitable intramedullary nails and systems are described in further detail in U.S. Patent No. 11,045,242.

The intramedullary nail 12 may be comprised of titanium, stainless steel, cobalt chrome, carbon composite, plastic or polymer-such as polyetheretherketone (PEEK), polyethylene, ultra high molecular weight polyethylene (UHMWPE), resorbable polylactic acid (PLA), polyglycolic acid (PGA), combinations or alloys of such materials or any other appropriate material that has sufficient strength, while also having sufficient biocompatibility to be implanted into a body. Similarly, the lag screws 14 or other bone fasteners may be comprised of titanium, cobalt chrome, cobalt-chrome-molybdenum, stainless steel, tungsten carbide, combinations or alloys of such materials or other appropriate biocompatible materials. Although the above list of materials includes many typical materials out of which intramedullary nails and bone fasteners are made, it should be understood that intramedullary nails and fasteners comprised of any appropriate material are contemplated.

A nail insertion instrument 30 may be used to install the nail 12 and align one or more of the bone anchors 14 to the respective openings 24, 26 in the nail 12. In some embodiments, the nail insertion instrument 30 may have an implant holder 31 with an implant holder tip or coupling portion 32, a handle grip or handle portion 34, and an aiming arm 36. The implant holder 31, handle portion 34, and aiming arm 36 may be separate parts or may be formed of a single, integrally formed component. The implant holder 31 with coupling portion 32 releasably engages or couples to the proximal portion 20 of the nail 12. For example, the free end of the coupling portion 32 may have a snap-fit design or other suitable mechanism to temporarily retain the intramedullary nail 12 for installation.

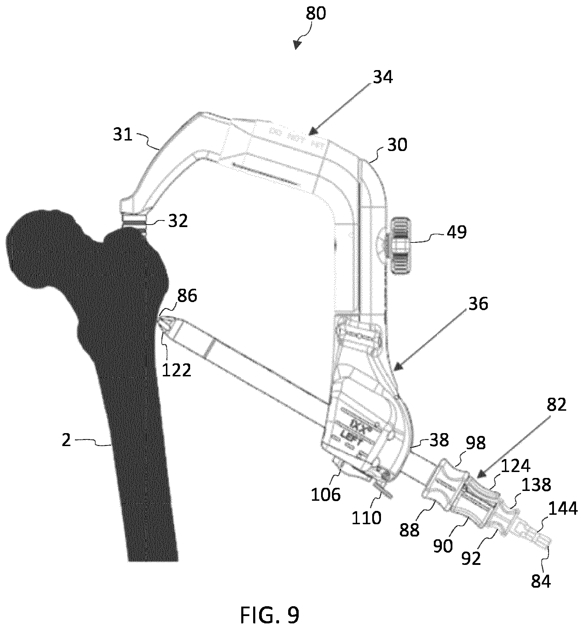

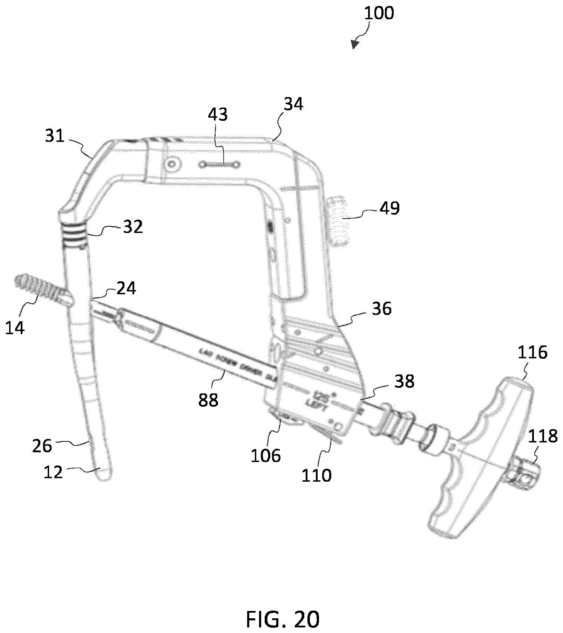

Depending on the instrument, the nail insertion instrument 30 may have a generally arcuate or J-shaped body. The handle portion 34 may include a hand grip and lower aiming arm 36 with one or more openings 38 configured to guide the bone anchors 14 into position. Each opening 38 may have a hole axis that substantially aligns with a corresponding hole axis of the respective nail openings 24, 26. The openings 38 may be configured to receive a sheath or sleeve (as further described in FIGS. 9 and 20) configured to protect soft tissue and allow for insertion of a drill, driver, K-wires, fasteners, and the like configured to be aligned with and inserted through the openings 24, 26 in the intramedullary nail 12 and into the bone. The nail insertion instrument 30 may include attachment locations for one or more modules (e.g., recon module, oblique module) for guiding additional fasteners 14 into the nail 12. Additional details of suitable nail insertion tools and assemblies are described in further detail in U.S. Patent No. 11,045,242.

### Trochanteric Nail Anteversion Alignment Systems

Trochanteric nails 12 may be inserted into the canal of the femur 2 through the greater trochanter to stabilize a variety of proximal femur fractures. Femoral neck anteversion is the angle that describes the twist that is present in the femur 2 between the hip and the knee. During insertion of the trochanteric nail 12, in order to ensure that the lag screw 14 is centered through the femoral neck and head, the surgeon matches the natural anteversion angle of the patient. Due to the minimally invasive nature of the trochanteric nailing procedure, this anteversion alignment may be performed under x-ray guidance.

Turning now to FIGS. 1-5, an anteversion alignment system 40 is shown according to one embodiment. The anteversion alignment system 40 is configured for aligning the trochanteric nail 12 to the optimal anteversion angle prior to lag screw 14 insertion. The anteversion alignment system 40 is integrated into the aiming arm 36 and/or nail insertion handle 34. The anteversion alignment system 40 includes an anteversion guide wire or K-wire hole 42 and radiopaque goal posts 44.

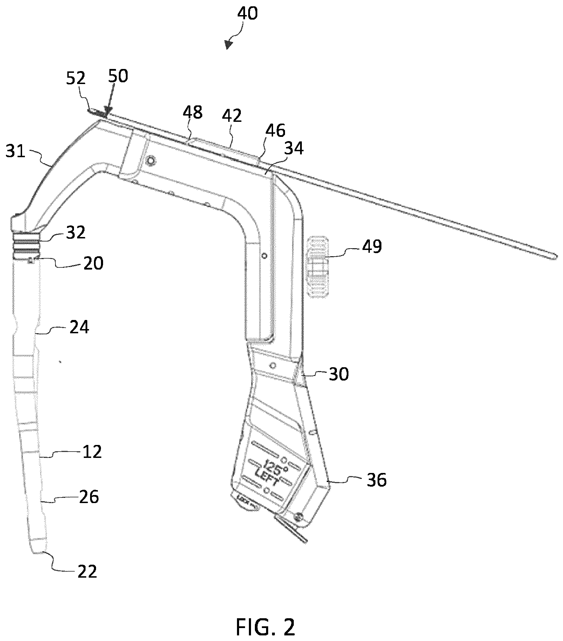

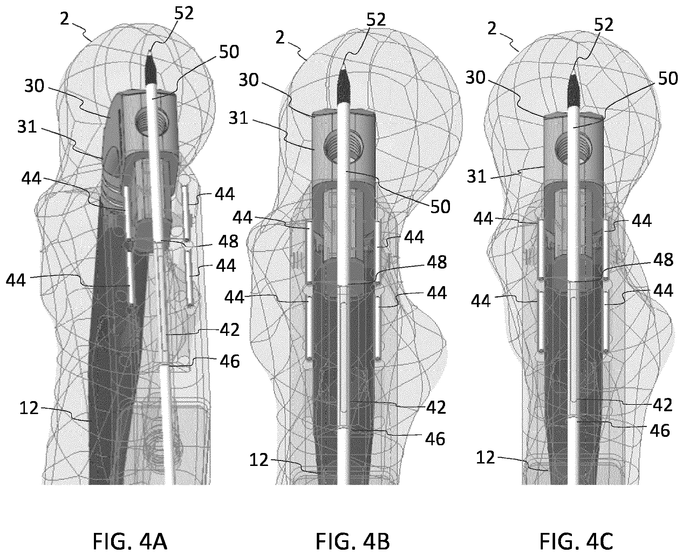

As best seen in FIG. 2, the anteversion guide wire or K-wire hole 42 is located on the top surface of the insertion handle 30 along the center plane of the instrument. The anteversion guide wire or K-wire hole 42 may be located in a projection or raised part on the top surface of the instrument 30. The anteversion guide wire or K-wire hole 42 may be a channel or pathway extending from a first or proximal end 46 to a second or distal end 48. The hole 42 is sized and dimensioned to receive a guide wire or K-wire 50 therethrough. The guide wire or K-wire 50, also known as a Kirschner wire, is a thin wire or pin with a distal tip 52 configured to temporarily engage bone. In this case, however, the anteversion K-wire 50 is utilized to indicate anteversion angle on imaging without being inserted into the patient. The wire 50 allows for visualization of the anteversion angle of the trochanteric nail 12. With this view, the position of the instrument 30 may be adjusted as necessary prior to insertion of the lag screw 14. As best seen in FIG. 4C, the optimal anteversion angle may be achieved when the wire 50 is centered through the femoral neck and head of the femur 2 on a lateral x-ray.

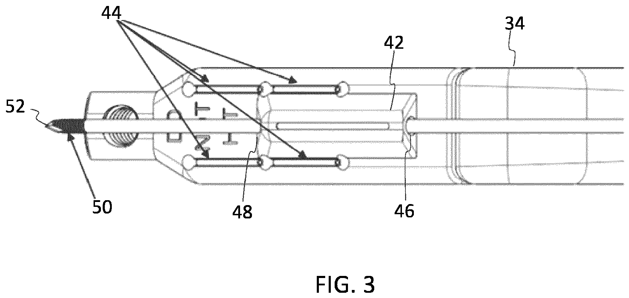

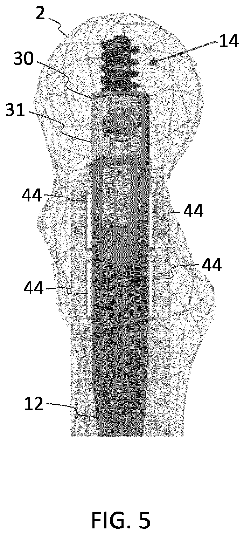

As best seen in FIG. 3, the goal posts 44 may include radiopaque goal posts 44 positioned on opposite sides of the K-wire 50. In one embodiment, the radiopaque goal posts 44 include two parallel rows of pins made from a radiopaque material, such as stainless steel, titanium, tungsten or other materials that are visible on imaging. The goal posts 44 may be aligned as two parallel rows of pins retained in the handle 34, for example, a pair of pins on each side. The goal posts 44 are aligned parallel to the center plane of the insertion handle 30 and spaced apart, for example, to generally match the outer diameter of the intramedullary nail 12. Under x-ray, the radiolucent carbon fiber handle 34 appears translucent while the goal posts 44 appear opaque and are clearly on the radiographic images. The goal posts 44 function as an alignment aid when aligning the C-arm with the insertion handle 30 on the lateral x-ray. The objective is to align the C-arm to be parallel with the center plane of the handle 30. Optimal alignment is achieved when the nail 12 is centered between the goal posts 44 as best seen in FIG. 5.

FIGS. 4A-4C detail one workflow for aligning the anteversion system 40. FIG. 4A shows a view of the nail 12 inserted into femur 2 with anteversion system 40 in which the C-arm is misaligned with the insertion handle 30. In this view, the anteversion system 40 and attached nail 12 appears tilted, the K-wire 50 is not centered on the nail 12, and the goal posts 44 are not parallel with one another or aligned with nail 12. When the C-arm is adjusted to the optimal trajectory, the goal posts 44 become centered, parallel around the nail 12 as shown in FIG. 4B. However, FIG. 4B still shows a misalignment of the nail anteversion because the K-wire is misaligned with the neck and head of the femur. Rotational adjustment of the nail 12 to the optimal anteversion is achieved when the K-wire 50 is centered through the femoral neck and head as shown in FIG. 4C. In this view, the implant holder 31 and attached nail 12 appear straight and vertically aligned, the K-wire 50 is centered through the femoral neck and head, and the goal posts 44 are aligned parallel to one another. Once the optimal anteversion is found, the lag screw 14 may be positioned into nail 12 via handle 34 and aligned into the body of the nail 12. Through this workflow, the anteversion alignment system 40 enables placement of the lag screw 14 at the optimal anteversion angle as shown in FIG. 5.

FIG. 5 shows a lateral view of the femur 2, intramedullary nail 12, handle implant holder 31 with the K-wire 50 removed, and lag screw 14. As shown, the lag screw 14 is inserted into the intramedullary nail 12 with optimal anteversion angle and placement in the femoral neck and head. The anteversion alignment system 40 requires minimal instrumentation and setup to use. The alignment system 40 utilizes goal posts 44 that are built into the nail insertion handle 34 and a single K-wire 50, which is readily available during all trochanteric nailing procedures. In addition, the anteversion alignment system 40 is non-invasive for the patient since the anteversion K-wire 50 is positioned to indicate anteversion angle on x-ray without being inserted into the patient.

### Trochanteric Nail Anti-Rotation Systems

Trochanteric nails 12 may be positioned within the femur's canal via the greater trochanter, ensuring stability for numerous types of fractures located at the proximal end of the femur 2. In certain fractures, especially in basicervical fractures, which occur at the junction of the femoral neck and trochanteric region, there is less rotational stability in the hip fracture. In such fractures, the torque required to insert the lag screw 14 through the femoral neck and head can cause the femoral head to spin. As such, the surgeon may need to stabilize the fracture to prevent this rotation during lag screw insertion.

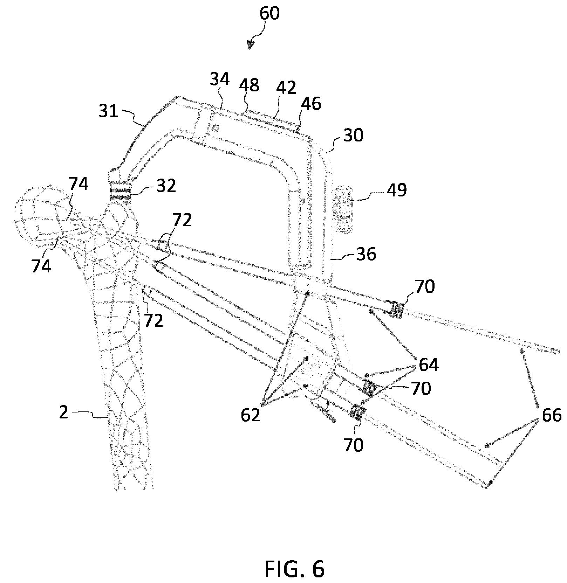

Turning now to FIGS. 6-8D, an anti-rotation system 60 is shown according to one embodiment. The anti-rotation system 60 is configured to stabilize and prevent rotation of the femoral head when the lag screw 14 is inserted and rotated through the nail 12 and into the neck and head of the femur 2. The anti-rotation system 60 is integrated into the aiming arm 36 and/or nail insertion handle 34. The anti-rotation system 60 may include anti-rotation sleeve holes 62 built into the carbon fiber aiming arm 36, anti-rotation sleeves 64 positionable through the sleeve holes 62, and anti-rotation guide wires 66 positionable through the anti-rotation sleeves 64.

As best seen in FIG. 6, the anti-rotation system 60 may include a plurality of anti-rotation sleeve holes 62 passing through the nail insertion handle 34. The holes 62 are sized and dimensioned to receive respective anti-rotation sleeves 64. The anti-rotation holes 62 may be built directly into the carbon fiber aiming arm 36. Therefore, no additional instrumentation or assembly is required. The sleeves 64 may be tissue protection sleeves configured to provide a guided pathway for the insertion of the anti-rotation guide wires 66 into bone, while minimizing damage to the surrounding soft tissues. The sleeve 64 extend from a first or proximal end 70 outside the handle 30 to a second or distal end 72 adjacent to the bone. The sleeve 64 may be a hollow tube defining a channel sized and dimensioned to receive the guide wire 66 therethrough. The guide wire 66 may be a Kirschner wire (K-wire) or other thin wire or pin with a distal tip 74 configured to temporarily engage bone. The distal tip 74 may be threaded to provide additional stability and anchorage within the bone.

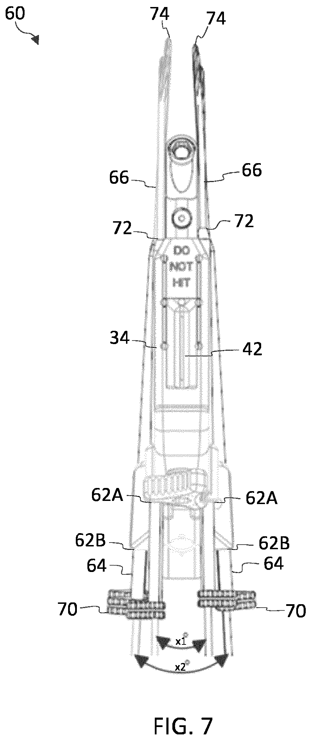

Turning now to FIG. 7, the anti-rotation system 60 may include up to six anti-rotation options, with three pathways on the left side of the aiming arm 36 and three pathways on the right side of the aiming arm 36. The anti-rotation system 60 may include an upper pair of holes 62A with one on the left side and one on the right side and a lower set of holes 62B with two on the left side and two on the right side. The upper pair of holes 62A may be located through the body of the aiming arm 36 above the set of lower holes 62B. The upper pair of holes 62A may converge distally at an angle x1 and the lower set of holes 62B may converge at an angle x2, which is greater than angle x1. In one embodiment, angle x1 is 0.8° angle with a trajectory that is 15° offset from the axis of the lag screw 14 in the A/P view. The lower set of holes 62B may have axes that are placed 10mm above and 10mm below the lag screw axis and aim parallel to the lag screw 14 in the A/P view. The lower set of holes 62B may converge where angle x2 is a 3° angle. Although specific locations and angles are shown, it is contemplated that other suitable pathways may be used. The surgeon may use any or all of the six anti-rotation options to help stabilize bone fragments in the proximal femur. The converging trajectories allow the anti-rotation guide wires 66 to consistently miss the intramedullary nail 12 and stay within bone through the femoral neck and head. The converging anti-rotation sleeve trajectories may provide added benefits. For example, with the converging trajectories, the anti-rotation wires 50 are more likely to stay within bone when advanced through the femoral neck and head. In addition, the converging angle may help to prevent skiving and deflection of the wires 50 away from the bone during initial insertion.

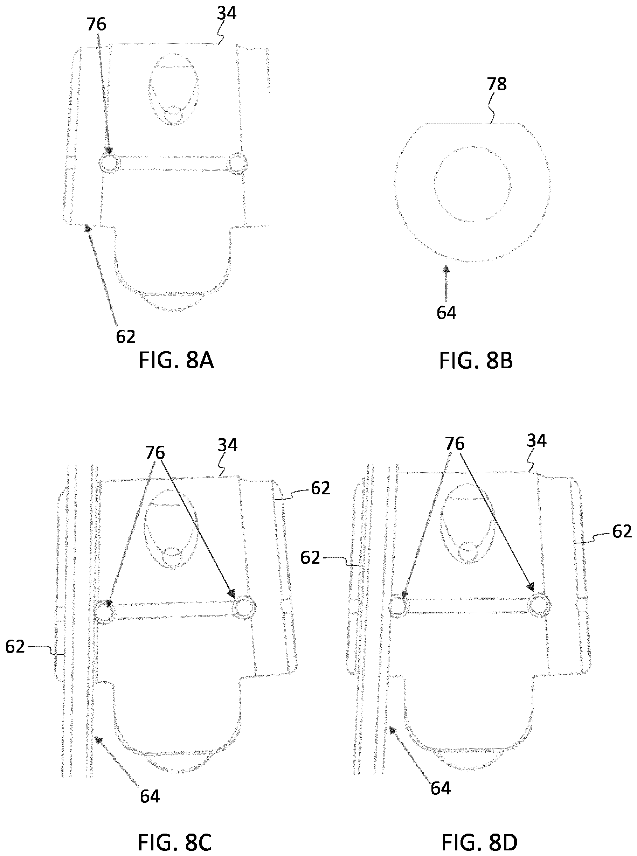

With further emphasis on FIGS. 8A-8D, the anti-rotation system 60 may include retention pins 76 configured to secure the anti-rotation sleeves 64 in the anti-rotation holes 62. Retention of the sleeves 64 prevents the sleeves 64 from moving or falling out during use. As shown in FIG. 8A, the retention pins 76 may include PEEK retention pins positioned to interfere with the anti-rotation holes 62. For example, one retention pin 76 may be positioned along one side of each anti-rotation hole 62. As shown in FIG. 8B, the anti-rotation sleeve 64 may have a D-shaped shaft which interfaces with the PEEK retention pins 76. The D-shaped shaft of sleeve 64 has a cross-sectional profile resembling a D shape where a portion of the shaft defines a flat side 78. When the sleeve 64 is inserted in the unlocked position shown in FIG. 8C, the flat 78 in the D-shaped shaft is clear of the PEEK pin 76. When the sleeve 64 is rotated to the locked position shown in FIG. 8D, the shaft interferes with and deflects the PEEK pin 76 engaging the friction fit retention of the sleeve 64. The friction fit engagement eliminates all toggle and clearance between the hole 62 and the sleeve 64. This reduces the risk of sleeve deflection, improving wire placement accuracy. Thus, the retention pins 76 prevent the anti-rotation sleeves 64 from moving or falling out and minimize skiving/deflection of the anti-rotation guide wires 50 during insertion.

### Trochanteric Nail Lag Screw Sleeve Assembly

Trochanteric nails 12 may be placed within the femoral canal via the greater trochanter to secure multiple types of fractures in the upper part of the femur 2. The trochanteric nail 12 may utilize a lag screw 14 which is inserted through the proximal diameter of the nail 12 into the femoral head. Prior to lag screw drilling and insertion, the lag screw sleeves and trocar are preferably inserted down to bone. Once inserted, these sleeves act as a baseline for guide wire insertion, measurement for lag screw length, lag screw drilling and lag screw insertion. In order to establish a reliable and accurate measurement baseline, it is important that the lag screw sleeves are inserted all the way to the bone. Advancing these large sleeves through soft tissue can cause resistance and difficulty for users during insertion.

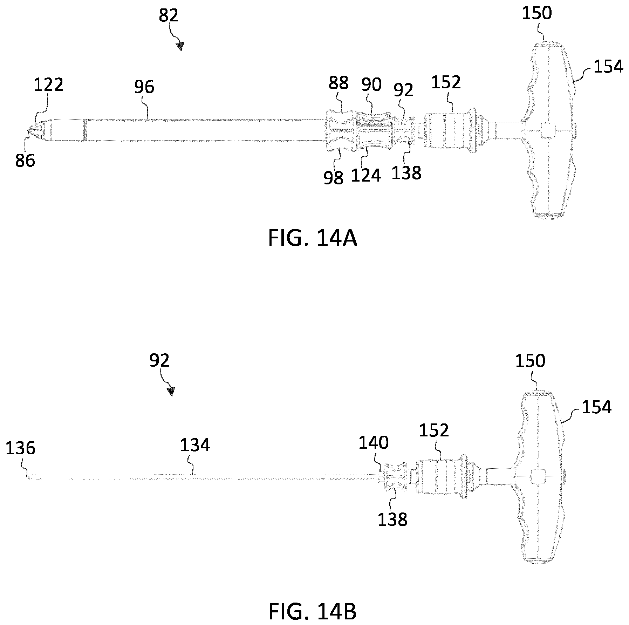

Turning now to FIGS. 9-16, a lag screw aiming assembly 80 is shown according to one embodiment. The lag screw aiming assembly 80 includes a lag screw sleeve assembly 82 configured to enable surgeons to reliably insert the sleeve down to bone during the trochanteric nailing procedure. The lag screw sleeve assembly 82 is compatible with the aiming arm 36 of nail insertion instrument 30. The lag screw sleeve assembly 82 extends from a proximal end 84 configured to interface with a handle (e.g., as shown in FIGS. 14A) to a distal end 86 configured to contact bone. As shown in FIG. 9, once the trochanteric nail 12 is inserted into the femur 2 at the desired depth and the aiming arm 36 is attached to handle 34, the lag screw sleeve assembly 82 is inserted through the aiming arm 36.

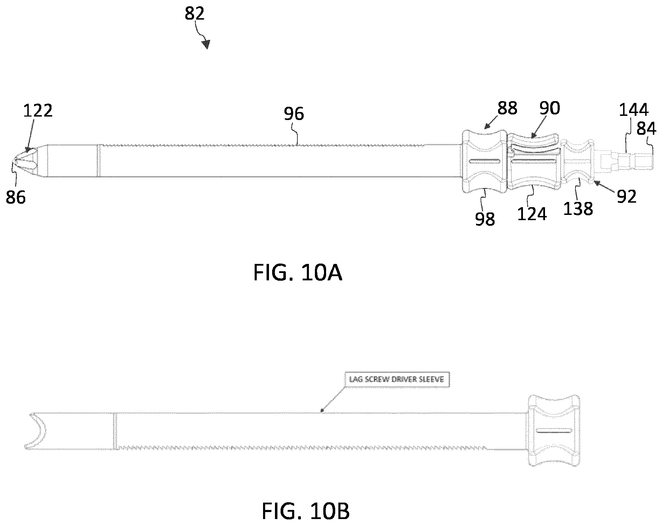

As best seen in FIGS. 10A and 10B, the lag screw sleeve assembly 82 includes a lag screw driver sleeve 88, a lag screw wire sleeve 90, and a lag screw trocar 92. The lag screw driver sleeve 88 acts as the outer sleeve for this assembly as well as a cannula for the lag screw step drill, the lag screw tap, and the lag screw driver. The lag screw driver sleeve 88 defines a tubular body with a hollow central through channel 94 and an outer surface defining a plurality of notches or teeth 96 configured to engage with a ratchet 104 or catch mechanism within the aiming arm 36. The ratchet mechanism acts as a retention feature for the driver sleeve 88. The proximal end includes an enlarged head portion 98, which may act as a stop for the lag screw wire sleeve 90. The lag screw driver sleeve 88 advances through and retains within the aiming arm 36.

FIG. 10B provides a lag screw driver sleeve with an extended distal end with a beveled tip. This beveled tip provides a more streamlined profile to facilitate insertion through soft tissue. Additionally, this beveled tip enables clear visualization of the proximal end of the lag screw during insertion into the femoral head. This allows the surgeon to optimize the insertion depth of the lag screw under X-ray guidance. The lag screw driver sleeve also provides a measurement baseline at the distal end of the driver sleeve for lag screw length measurement. The sleeve assembly is inserted so the measurement baseline feature on the lag screw driver sleeve contacts bone. Then the lag screw wire is inserted under x-ray to an optimal depth within the femoral head as determined by the surgeon. The wire must then be measured to determine the optimal lag screw length.

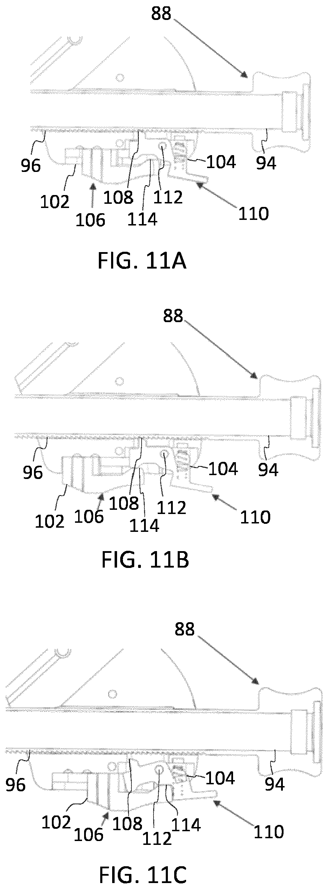

With further emphasis on FIGS. 11A-11C, advancement and retention of the lag screw driver sleeve 88 may be facilitated by a ratchet assembly 102 within the aiming arm 36. The ratchet assembly 102 may include a ratchet 104 configured to interface with the notches or teeth 96 on the driver sleeve 88 and a ratchet slide lock 106 configured for locking/unlocking the ratchet 104. The ratchet assembly 102 enables the user to quickly engage and disengage the ratchet 104 to easily insert and remove the driver sleeve. When engaged, the ratchet 104 may permit linear motion in one direction to provide controlled unidirectional movement, preventing backward motion. The ratchet 104 may include a pawl 108 that interacts with the teeth 96 of the sleeve 88. As the pawl 108 advances over the teeth 96, the sleeve 88 moves in the permitted direction, and prevents the sleeve 88 from moving backward. The ratchet 104 may be a pivoting component to allow the pawl 108 to be engaged and disengaged from the teeth 96. The ratchet 104 may pivot about a fixed pivot pin 112 when the user depresses a thumb lever or paddle 110. The ratchet 104 may be spring-loaded such that the ratchet 104 returns to the engaged position (e.g., as shown in FIG. 11A) when the paddle 110 is released.

As shown in FIG. 11A, when the ratchet 104 is engaged, the sleeve 88 is free to advance through the aiming arm 36 through soft tissue but is locked from moving in the reverse direction away from the patient. This mechanism enables surgeons to reliably advance through soft tissue without having the driver sleeve 88 back out as a result of soft tissue forces. As shown in FIG. 11C, the user may disengage the ratchet 104 from the ratchet teeth 96 on the driver sleeve 88 to adjust the sleeve position using the ratchet paddle 110. As shown in FIG. 11B, once the driver sleeve 88 has been fully inserted, the ratchet slide 106 can be advanced forward to lock the driver sleeve 88 in place in both directions. In this configuration, an arm 114 of the slide 106 abuts the base of the ratchet 104, thereby preventing the ratchet 104 from pivoting and disengaging. This locked position sets a rigid baseline position for the remainder of the lag screw insertion process. Once the lag screw 14 is inserted in the nail 12 and bone, the ratchet slide lock 106 can be pulled back to the un-locked position, to disengage the ratchet 104 and facilitate easy sleeve removal.

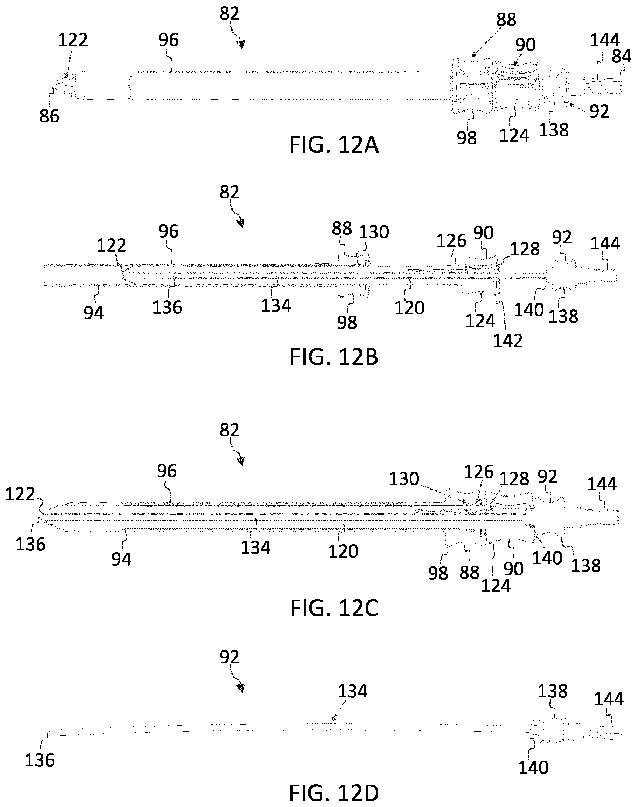

With further emphasis on FIGS. 12A-12D, the lag screw wire sleeve 90 is inserted into and retained by the lag screw driver sleeve 88 and acts as a guide sleeve for the lag screw guide wire and trocar 92. The lag screw wire sleeve 90 defines a tubular body with a hollow central through channel 120 sized and dimensioned to receive the guide wire 134. The distal end of the wire sleeve 90 may include a distal tip with cutting flutes 122, which facilitate easy insertion as the wire sleeve 90 is pressed and twisted through soft tissue. The proximal end of the wire sleeve 90 may include an enlarged head portion 124, which may rest against head 98 of the driver sleeve 88 when fully inserted and acts as a stop for the lag screw trocar 92.

The wire sleeve 90 may be retained in the driver sleeve 88, for example, with a spring tab 126. The wire sleeve 90 defines a spring cut 128 that enables it to snap into the driver sleeve 88. For example, the spring cut 128 may extend along the shaft of the wire sleeve 90 and through the head portion 90. As the wire sleeve 90 is inserted through driver sleeve 88, the spring tab 126 is deflected, allowing the wire sleeve 90 to pass through. When the spring tab 126 reaches an undercut 130 in the driver sleeve 88, the tab 126 snaps into place and locks the wire sleeve 90 from backing out but allows the wire sleeve 90 to spin freely. The retention of the wire sleeve 90 in the driver sleeve 88 maintains a rigid assembly between these components but allows the wire sleeve 90 to rotate as needed.

The lag screw trocar 92 is retained within the wire sleeve 90. The trocar 92 may include a shaft 134 with a sharp distal tip 136 configured to facilitate insertion of this assembly through soft tissue. As best seen in FIG. 12D, the trocar 90 may define a slight bend in its shaft 134. The bent trocar shaft 134 facilitates retention within the wire sleeve 90. For example, the bent shaft 134 may provide a friction fit retention within the wire sleeve 90 when inserted to prevent the trocar 92 from dropping out of the assembly 82. A proximal portion of the trocar 90 may include an enlarged head portion 138, which may rest against head 124 of the wire sleeve 90 when fully inserted. A base of the head portion 138 may include a mating member 140 configured to interface with a corresponding member 142 of the wire sleeve 90. For example, the mating members 140, 142 may include a male extension or protrusion with a corresponding female recess. In one embodiment, the mating members 140, 142 include a male hex on the trocar 92 and a female hex on the wire sleeve 90. When the trocar 92 is fully inserted into the wire sleeve 90 and the mating members 140, 142 are seated together, these instruments 90, 92 can turn together to help the assembly drive through soft tissue. The proximal end of the trocar 90 may include a handle interface 144 configured to secure a handle 150, such as a quick connect handle.

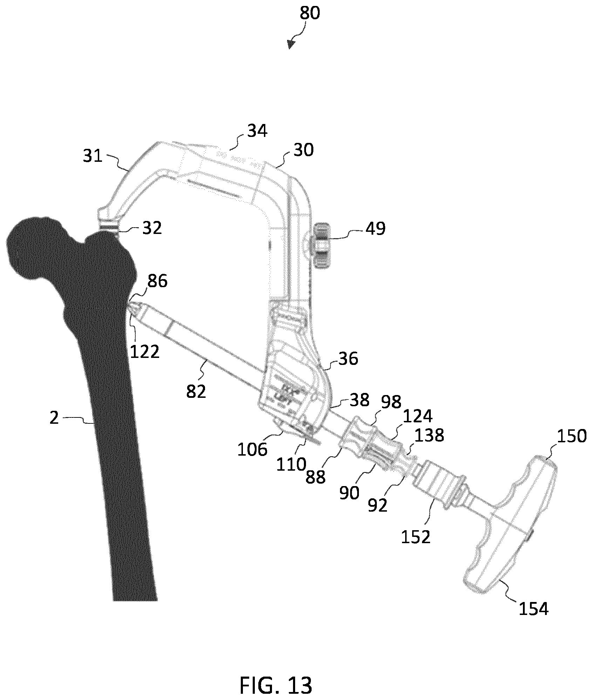

FIGS. 13-14B shows the lag screw aiming assembly 80 with handle 150 attached to the trocar 90. The handle 150 may be a quick connect handle with a collar 152 and a grip 154. The quick connect handle 150 provides the surgeon with additional leverage to advance through soft tissue. The collar 152 may include a quick attachment mechanism, such as push-and-click, twist-and-lock, or other fastening systems. The handle grip 154 may include a T-shape with a horizontal grip attached to a vertical shaft or other suitable ergonomic designs. The attachment of handle 150 provides greater leverage for the surgeon to press and twist the assembly through soft tissue until the cortex of the femur is reached. The cutting flutes 122 at the distal tip of the wire sleeve 90 and the sharp trocar tip 136 enable the surgeon to press and twist the sleeve assembly 82 to advance through soft tissue.

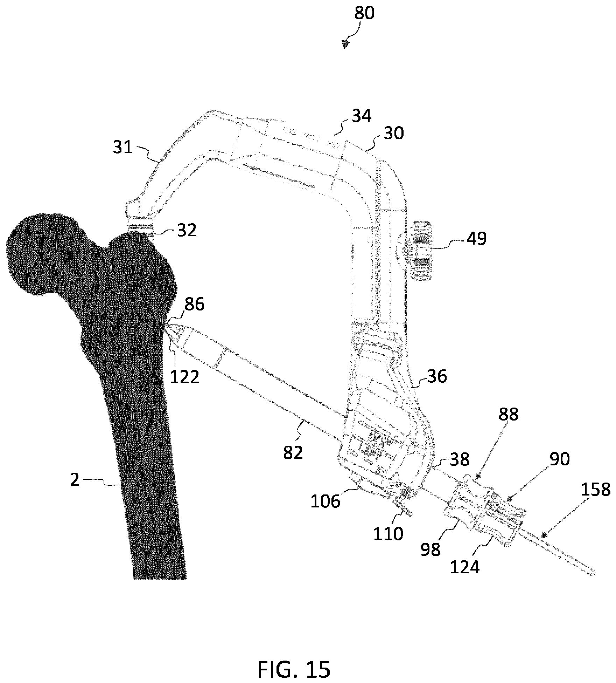

With emphasis on FIG. 15, after the trocar 92 is removed, a lag screw guide wire 158 may be passed through the lag screw wire sleeve 90. The lag screw sleeve assembly 82 facilitates the insertion and measurement of the lag screw guide wire 158. Once the sleeve assembly 82 is inserted to bone, the lag screw wire 158 may be inserted under x-ray or other imaging to an optimal depth within the femoral head as determined by the surgeon. The wire 158 may then be measured to determine the optimal lag screw length.

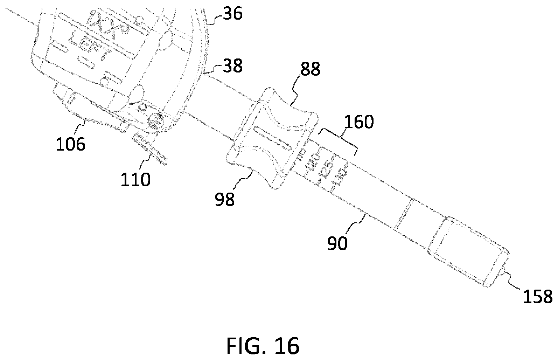

FIG. 16 shows a calibrated wire sleeve 90 configured to measure lag screw guide wire 158. The calibrated wire sleeve 90 may include a plurality of markings 160, such as graduated markings. The markings 160 may be etched or printed onto the outer surface of the wire sleeve 90, for example, with points, lines, numbers, or other visual symbols to signify a depth or length of the wire 158. The calibrated wire sleeve 90 may be used to quickly take the measurement without the need for additional measurement instruments. To take the measurement, the wire sleeve 90 may be disengaged from the driver sleeve retention and pulled back to be flush with the proximal tip of the guide wire 158. At this point, the calibrations may be read from the proximal face of the wire sleeve 90 where the end of driver sleeve 88 aligns with a given marking 160. For example, the measurement shown in FIG. 16 reads 115mm. In this manner, measurement using the sleeve assembly 82 enables fast and efficient measurement without additional instruments. Trochanteric nail procedures may occur very quickly, so any saved time is significantly valued by the surgeon.

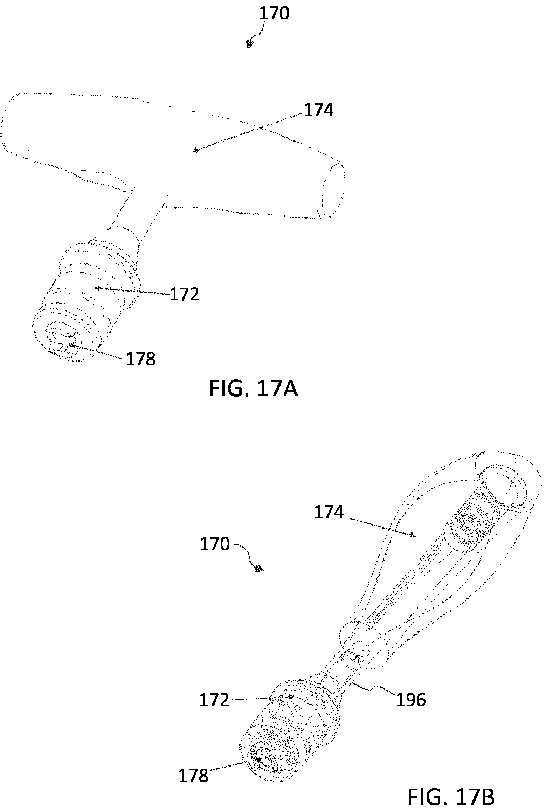

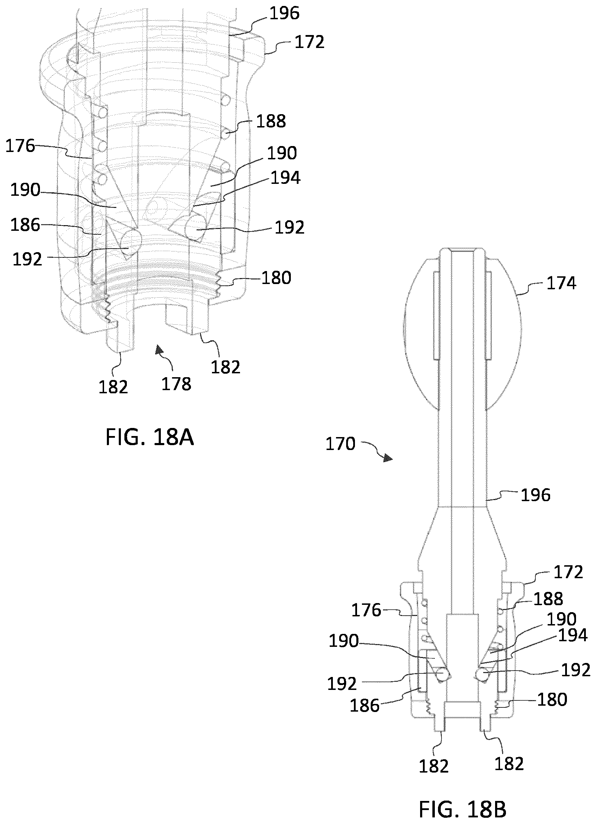

FIGS. 17A-19B show a push-connect handle assembly 170 according to one embodiment. Medical instruments use and support a variety of axial quick-connect male and female interfaces that allow modularity and cross-compatibility between instruments and handles and/or power systems. In some cases, female connections require a collar to be pulled back before inserting the male connection and releasing the collar to lock the male and female connections together rotationally and axially.

In this embodiment, the push to connect handle 170 removes the need for two handed operation of the quick connection upon assembly. Similar to handle assembly 150, push-connect handle assembly 170 may include a collar 172 configured for fast and secure connection of different instrument tips or attachments and a grip 174 configured to be held and manipulated by a user. The collar 172 may have a circular or cylindrical body define a central through opening 176 and a female connection 178 defined into the bottom of the collar 172. The female connection 178 may be configured for quick push-connect assembly to different instrument tips or attachments. As shown in FIG. 7A, the handle grip 174 may include a T-shape with a horizontal grip attached to a vertical shaft. In FIG. 7B, the handle grip 174 may include a vertical grip aligned with the tool's central axis. It will be appreciated that any suitable ergonomic grip 174 suited for securely holding the instrument may be used.

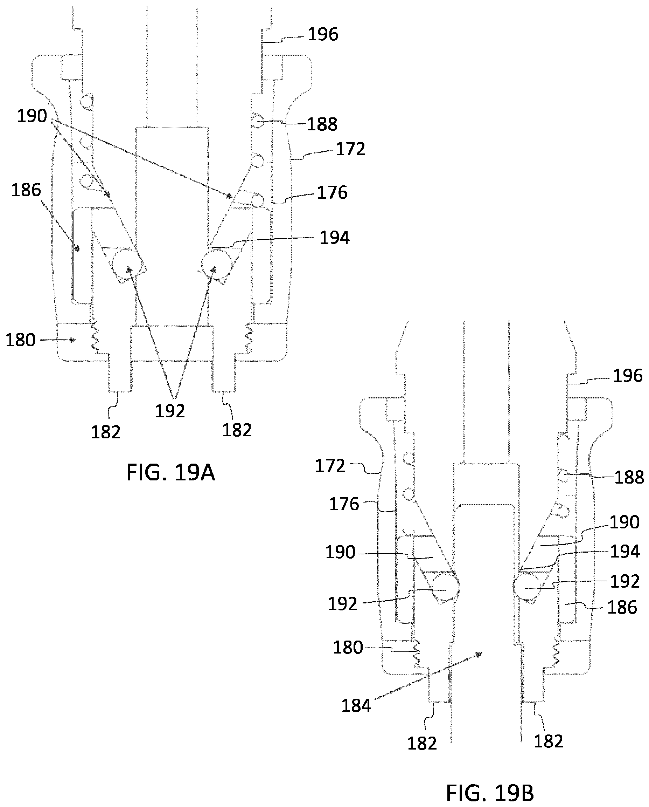

The collar 172 retains a threaded cap 180 with a pair of opposed arms 182 configured to retain a male portion 184 of the instrument tip. The male connection 184 may be a shaft with a free end receivable in female connection 178 of collar 172. In this embodiment, the male and female sides 178, 184 may be pushed together and locked without pulling back the collar 172. As best seen in FIGS. 19A-19B, an upper portion of cap 180 may be partially or fully encircled by a spring-loaded ring 186. The ring 186 may have a circular body sized and dimensioned to fit within the through opening 176 of collar 172. The ring 186 is biased downward via spring 188 positioned in an upper portion of opening 176.

A pair of slanted tracks or cuts 190 are configured to retain a pair of locking pins 192 therein. The slanted tracks or cuts 190 may be defined between a distal tip 194 of the shaft 196 of the handle 174 and an upper surface of the threaded cap 180. The slanted cuts 190 may be sloped or angled to point distally toward one another. The locking pins 192 may be configured to move along tracks 190 to retain the male end 184 of the connection. In particular, the male side 180 of the connection pushes back the locking pins 192 which ride on slanted cuts 190 and spring-loaded ring 186 which only allows insertion, but not removal once the locking pins 192 are sprung back into place. The quick connect locks the male and female connections 178, 184 together rotationally and axially. Removal may occur by pulling back of the collar 172 on the female connection 176. The ability to push to connect a number of handle types improves usability and reduces connection swapping time and frustration.

### Trochanteric Nail Proximal Aiming System

Trochanteric nails may be used to stabilize fractures of the proximal femur. A trochanteric nail proximal aiming system may be used by orthopedic surgeons to insert the trochanteric nail into a patient, insert the lag screw through the proximal end, and insert the distal locking screws for a short nail. The nail is first connected to the insertion handle and inserted into the femur after reaming is complete. The insertion handle allows the surgeon to advance, retract, and/or rotate the nail. The insertion handle is also used as a connection point for the aiming arm.

Turning now to FIGS. 20A-34, a proximal aiming system 100 is shown according to one embodiment. Proximal aiming system 100 is similar to lag screw assembly 80, where reference numerals refer to like elements. As best seen in FIG. 20, the aiming system 100 includes implant holder 31 with coupling portion 32, which is attachable to the proximal end of nail 12, handle portion 34 affixed to the implant holder 31, aiming arm 36 attached to the handle portion 34, and driver sleeve 88 extending through the aiming arm 36 to target the lag screw hole 24 in the nail 12.

The aiming arm 36 may be constructed of a composite carbon fiber material and may be used to target the proximal lag screw 14 and distal locking screw placement without fluoroscopy and associated radiation. The lag screw targeting hole 38 may include a ratcheting mechanism and ratchet lock, which is intended to control the insertion depth of the lag screw driver sleeve 88. Lag screw wire sleeve 90 and trocar 92 may be positioned respectively through the lag screw driver sleeve 88. The lag screw wire sleeve 90 may be retained by the lag screw driver sleeve 88 to allow placement of a K-wire through the lag screw hole 24 in the nail 12. The lag screw trocar 92 may be inserted through the lag screw wire sleeve 90 to aid in clearing soft tissue when targeting the lag screw hole 24 in the nail 12.

A lag screw driver 116 may be used to insert the lag screw 14 through the lag screw driver sleeve 88, and a lag screw retention rod 118 may be used to retain the lag screw 14 to the driver 116 during insertion or removal of the lag screw 14. The lag screw driver 116 and retention rod 118 are used to retain and insert the lag screw 14 through the lag screw driver sleeve 88. The locking screw target holes are intended to target the placement of a locking screw driver sleeve, wire sleeve, and trocar. The aiming system 100 also enables the surgeon to rotationally fix the femoral neck and head using the anti-rotation wire sleeves which target placement of a K-wire around the troch nail 12.

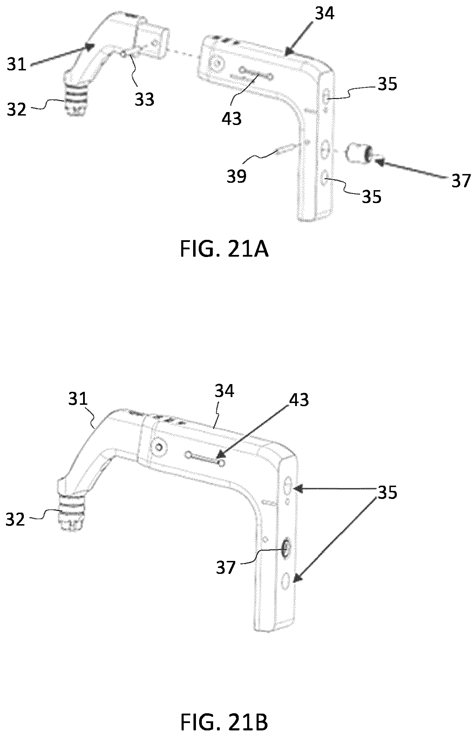

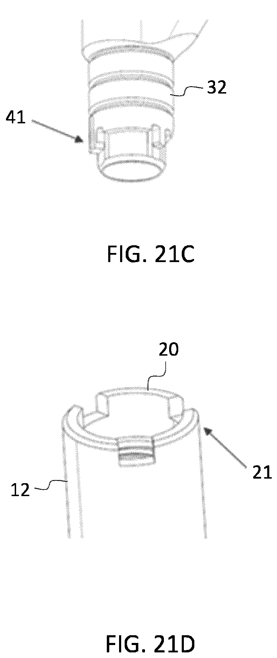

Turning now to FIGS. 21A-21D, the insertion handle assembly connects to and retains the nail 12 during nail insertion. The insertion handle assembly may include a carbon fiber composite material for the handle grip 34 mated to implant holder 31. The implant holder 31 may be press-fit into one end of the handle 34 and secured with pin 33, for example. The opposite end of the insertion handle 34 may have alignment holes 35 (e.g., a pair of locating pin holes) and a threaded insert 37 that is used to orient and connect the aiming arm 36. The alignment holes 35 and threaded insert 37 may be used to attach various troch nail aiming arms 36. The threaded insert 37 may be retained in handle 34 via pin 39 or in another suitable manner. The insertion handle 34 may include one or more radiographic marker pins 43 visible under x-ray or other imaging. The radiographic marker pins 43 may help to guide the placement of the nail 12 under x-ray, thereby enabling the surgeon to position the implant 12 more accurately.

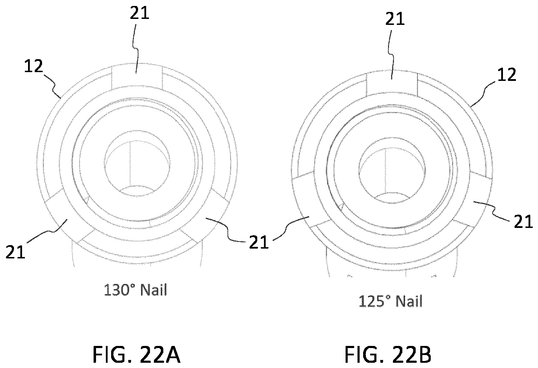

The implant holder tip or coupling portion 32 may be a stainless-steel shaft that protrudes from the handle grip 34 and acts as a connection point for the nail 12. The shaft of the coupling portion 32 defines an internal thread to retain the connection bolt 270 that is used to connect the nail 12 to the insertion handle 34. In one embodiment, the implant holder tip or coupling portion 32 may be a keyed implant holder tip configured to mate with both the 125° and 130° nails in a single orientation. As best seen in FIG. 21C-21D, the implant holder tip 32 may include a keyed interface 21, 41 which ensures that the nail 12 is connected in the correct orientation relative to the insertion handle 34. The stainless-steel shaft of the coupling portion 32 may have orientation tabs 41 configured to mate with corresponding recesses 21 in the proximal end 20 of the nail 12. For example, three equally spaced orientation tabs 41 may engage with respective recesses 21 to correctly position the nail 12 relative to the insertion handle 34. With further reference to FIGS. 22A-22B, the 125° and 130° nail implants 12 may have three keyed slots 21. The spacing of the keyed slots 21 may differ between these two nail types. The implant holder tip 32 may be configured to fit both of these nail tips in only a single orientation to ensure correct nail placement. Therefore, a single insertion handle 34 may be used for both nail types and ensures that the nails 12 may only be connected to the insertion handle 34 in the correct orientation for insertion and targeting.

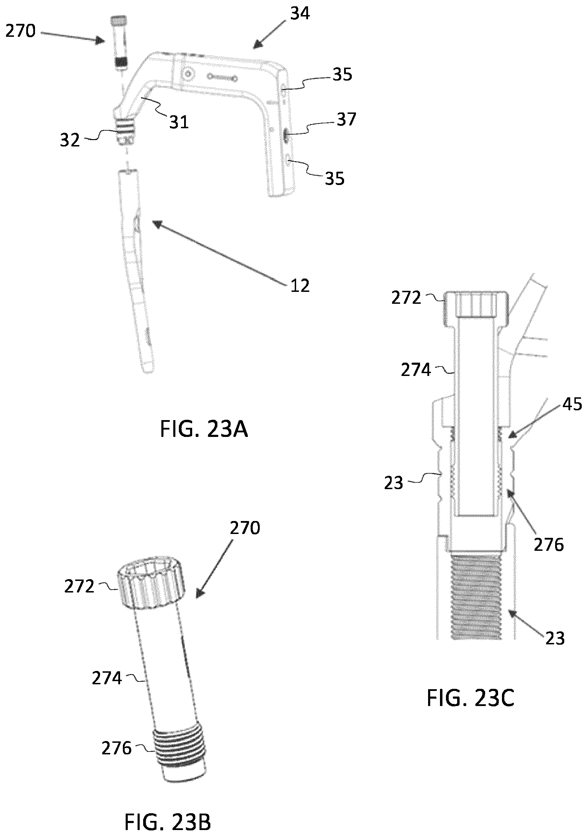

Turning now to FIGS. 23A-23C, nail connection bolt 270 is configured to secure the trochanteric nail 12 to the insertion handle 34. The cannulated nail connection bolt 270 may include a top or head 272, a cylindrical shank 274, and a lower threaded portion 276 configured to engage with a corresponding thread 23 in the nail 12. The external threads 276 on the nail connection bolt 270 pass through the internal threads 45 in the implant holder 31 to retain these parts together. The implant holder 31 is designed with internal threads 45 to retain the nail connection bolt 270, for example, to prevent the connection bolt 270 from dropping on the floor during use or pass offs. These same external threads 276 on the nail connection bolt 270 are then used to connect with the nail 12.

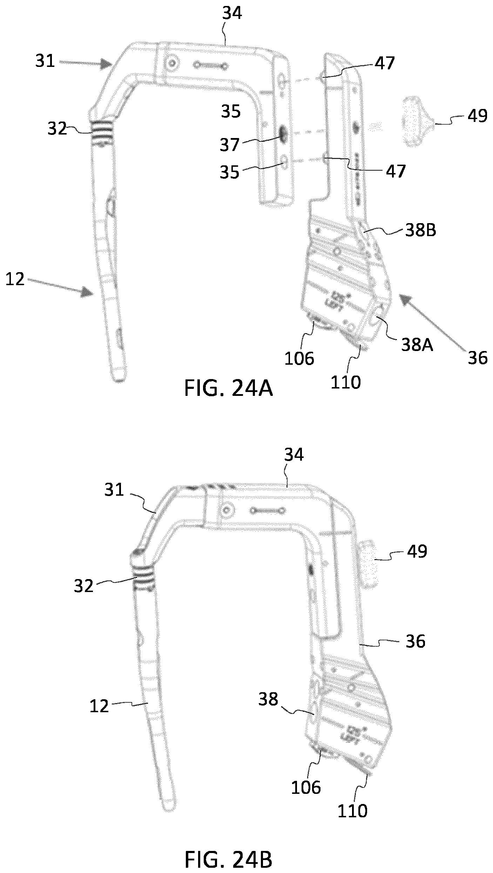

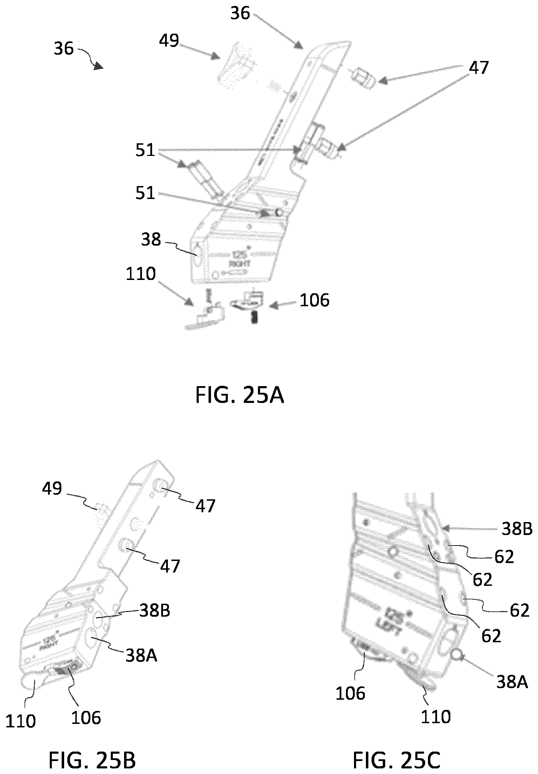

As shown in FIGS. 24A-24B and 25A-25C, alignment pins 47 and attachment knob 49 may be used to assemble the aiming arm 36 with the insertion handle 34. The system 100 may include two or more different aiming arms 36 intended to target the 125° and 130° nails 12. The aiming arm 36 may define one or more holes 38A, 38B with trajectories targeting the placement of the lag screw, the locking screw, and the anti-rotation wire sleeves. For example, hole 38A may target the proximal lag screw opening 24 and hole 38B may target the distal locking screw opening 26 in nail 12. One or more retention pins 51, such as PEEK retention pins, may be used to facilitate retention of the driver sleeves and anti-rotation wires during use. The ratchet mechanism 104 and ratchet lock 106 control the insertion depth of the lag screw driver sleeve 88.

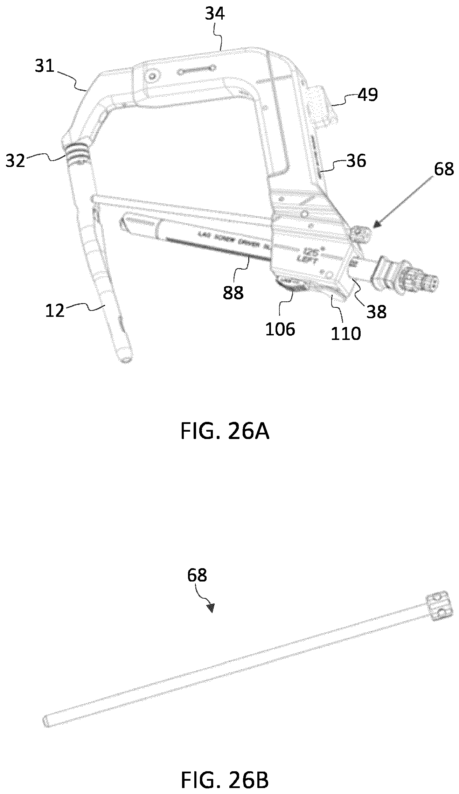

FIGS. 26A-26B show an anti-rotation wire sleeve 68 according to one embodiment. In this embodiment, the aiming arm 36 defines four holes 62 which target the placement of the anti-rotation wire sleeves 68. Guide wires or K-wires 66 may then be inserted through these sleeves 68 in order to prevent rotation of the femoral head during lag screw placement. These K-wires 66 may also be used to guide the insertion of cannulated screws into the femoral head on either side of the nail 12.

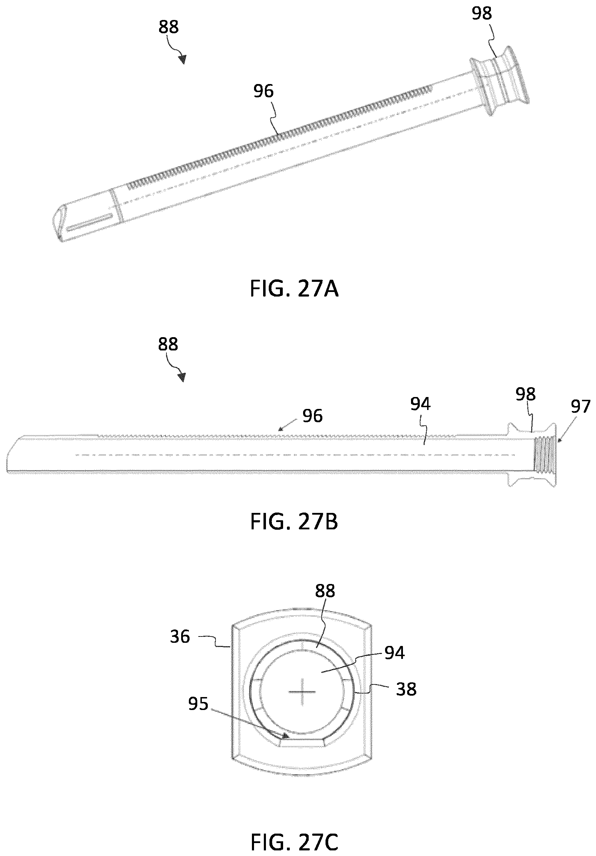

Turning now to FIGS. 27A-27C, the lag screw driver sleeve 88 is configured to be inserted through the aiming arm lag screw hole 38. The ratchet teeth 96 on the shaft interface with the aiming arm 36 to facilitate control of the insertion depth of the sleeve 88. In one embodiment, a flat side 95 of the shaft is configured to mate with a D-shaped hole 38 in the aiming arm 36 to ensure that the sleeve 88 is inserted in the correct orientation. In this embodiment, internal threads 97 in the sleeve 88 are configured to mate with corresponding external retention threads 91 on the wire sleeve 90, to retain the lag screw wire sleeve 90.

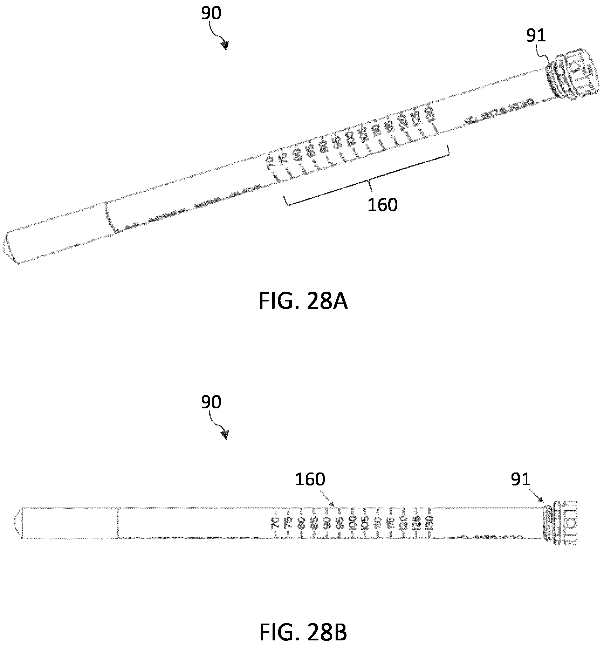

FIGS. 28A-28B show lag screw wire sleeve 90 with lag screw length calibrations according to one embodiment. The lag screw wire sleeve 90 is sized and dimensioned to be inserted into and retained by the lag screw driver sleeve 88. The wire sleeve 90 guides the insertion of both K-wires and the lag screw trocar 92. One or more markings 160, such as calibration markings, may be used to determine the lag screw length that is needed. In order to take this measurement, the wire sleeve 90 may be seated flush with the lag screw driver sleeve 88. The K-wire 158 is then inserted to the desired depth. The wire sleeve 90 is then pulled back so the proximal surface of the sleeve 90 is in line with the proximal tip of the K-wire 158. In this position, the proximal surface of the lag screw driver sleeve 88 indicates the necessary lag screw length. The lag screw wire sleeve 90 enables the user to use the calibrated markings 160 on the shaft to determine lag screw length without the use of a separate depth gauge. This reduces the number of instruments required and simplifies the workflow.

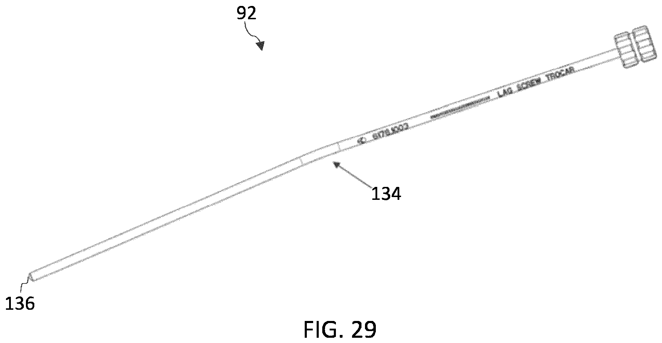

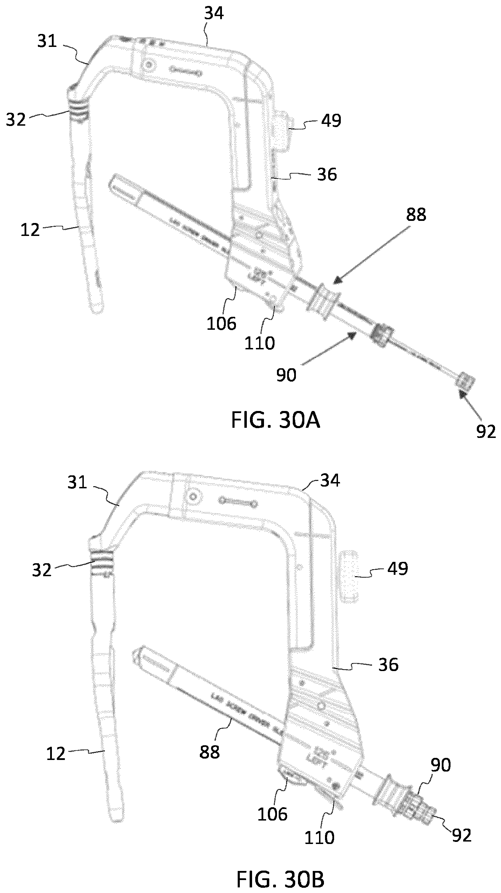

FIG. 29 shows the lag screw trocar 92 according to one embodiment. The lag screw trocar 92 is used to clear soft tissue as the lag screw driver sleeve 88 and wire sleeve 90 is inserted. In one embodiment, the trocar 92 may have a bent shaft 134, which facilitates retention of the trocar 92 within the lag screw wire sleeve 90. FIGS. 30A-30B show the lag screw driver sleeve 88 inserted through the aiming arm 36, the lag screw wire sleeve 90 positioned through the driver sleeve 88, and the lag screw trocar 92 positioned through the wire sleeve 90.

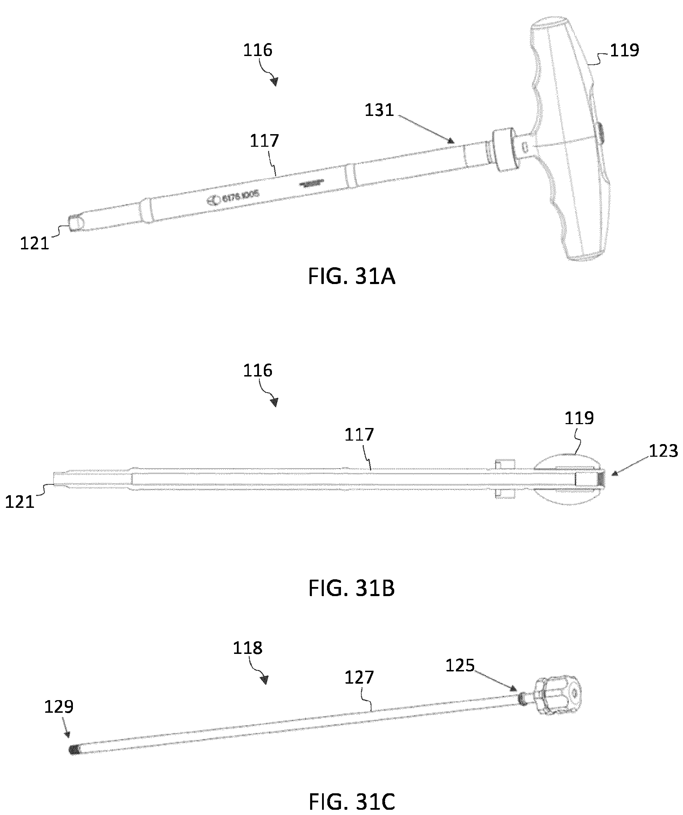

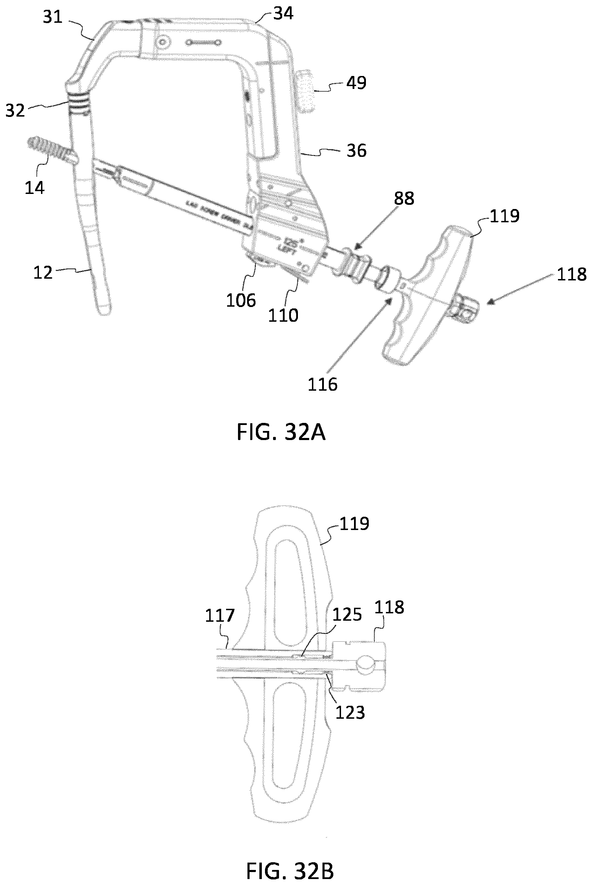

Turning now to FIGS. 31A-31C and 32A-32B, lag screw driver 116 and lag screw retention rod 118 are shown according to one embodiment. The lag screw driver 116 is used to insert the lag screw 14 through the lag screw driver sleeve 88. The lag screw retention rod 118 is inserted into and retained by the lag screw driver 88 to secure the lag screw 14 to the driver 116 during insertion or removal. The lag screw driver 116 includes a hollow shaft 117 and a handle 119, such as a T-shaped handle. The shaft 117 may provide a depth indicator marking 131 to indicate when the driver 116 reaches a desired depth relative to sleeve 88. The shaft 117 terminates at a distal tip 121, which interfaces with a corresponding recess in the lag screw 14. The proximal end of the shaft 117 defines inner retention threads 123, which interface with corresponding exterior threads 125 on the retention rod 118. The retention rod 118 includes a shaft 127 sized and dimensioned to fit through the driver shaft 117. A distal end of the shaft 127 includes connection threads 129 configured to interface with the lag screw 14. The lag screw retention rod 118 is inserted into and retained by the lag screw driver 116 using the retention threads 123, 125 on each part. The retention rod 118 threads past the internal threads 123 on the driver 116 and is retained. The distal connection threads 129 on the retention rod 118 retains the lag screw 14 to the driver 116 during insertion or removal of the lag screw 14.

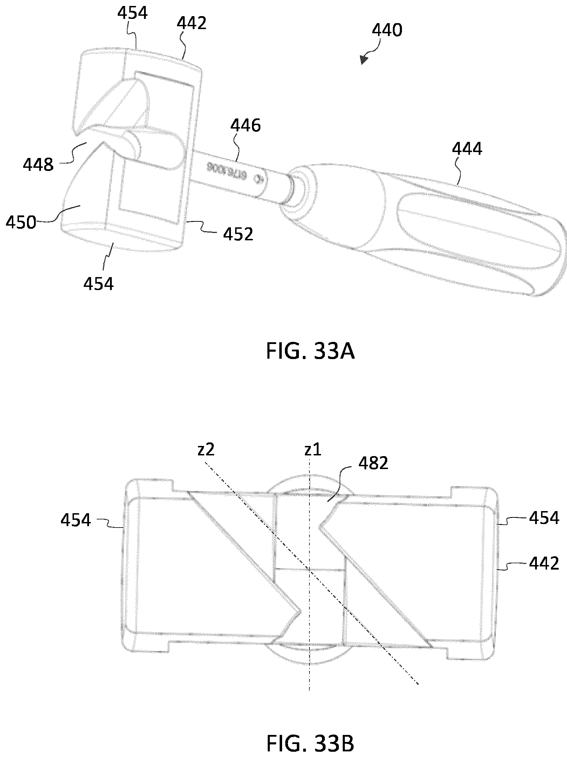

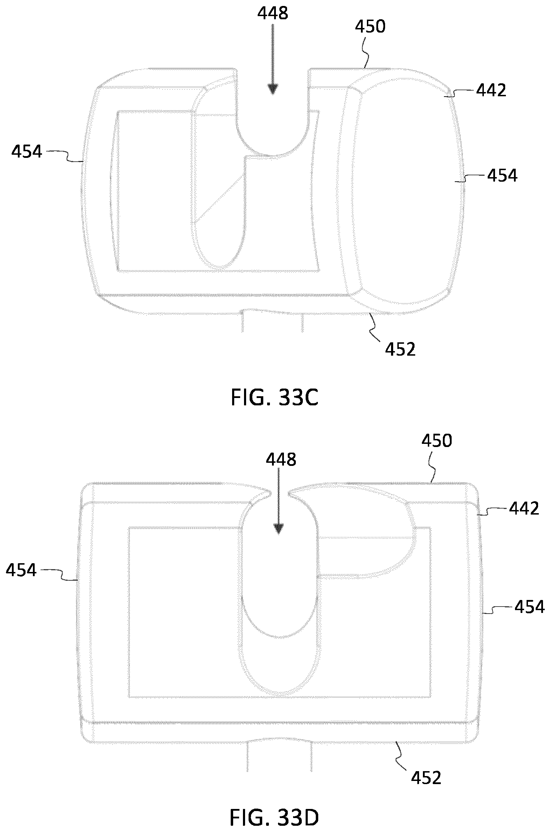

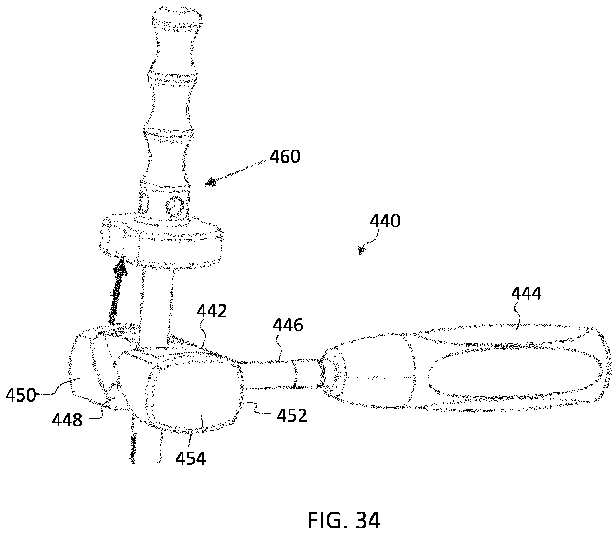

Turning now to FIGS. 33A-33D and 34, impaction mallet 440 may be used to insert and remove the nails 12. The mallet 440 includes a mallet head 442, a mallet handle 444, and a mallet shaft 446 connecting the head and handle 442, 444. The mallet head 442 may have a generally rectangular shape with a slot 448 defined therein. The mallet head 442 may have a top surface 450, an opposite bottom surface 452 with the shaft 446 extending therefrom, and opposed striking surfaces 454 for imparting an impaction force. The slot 448 is defined through the top surface 450 and may define a first portion along central axis z1 extending along a central plane through the instrument 440, and a second portion along diagonal axis z2 extending along a transverse plane. The bottom areas of slot 448 may be sloped or angled. The depth of the slot 448 along axis z1 may be deeper than the depth of slot 448 along axis z2. During insertion, the mallet 440 impacts an impaction rod, which is attached to the insertion handle 34 to drive the implant 12 into the femur. During removal, a slap removal shaft 460 may be used as shown in FIG. 34. In FIG. 33C, the slap removal rod 460 is inserted into slot 448 along axis z2. The mallet 440 is rotated 45° to the position shown in FIG. 33D and the rod 460 is seated into the deeper area of slot 448 along axis z1. The slot 448 encircles the removal shaft 460 and slides along the shaft 460 to impact the implant 12 out of the patient. The slot geometry is configured to retain the mallet 440 to the shaft 460 during slap removal. The retention slot 460 on the mallet 440 prevents the mallet 440 from slipping off of the slap removal rod 460 during impaction.

### Intramedullary Nail Coring Reamer Instrument

Intramedullary nails, such as trochanteric nails, are inserted into the canal of long bones via the axial ends of the bone (e.g., the piriformis fossa or greater trochanter for antegrade nails and trochanteric nails, respectively, and the intercondylar fossa for retrograde nails) to stabilize a variety of long bone fractures. Prior to nail insertion, access to the canal of the long bone may be opened at one of these axial ends by reaming of a dense cortical wall, for example, over an entry wire used to establish the starting point or hole. Opening reaming may be performed using a tapered, rigid reamer that functions using sharp side-cutting edges to shave away the bone and flutes that push the cut material out. When a fracture line is present at the preferred entry point, however, a tapered, rigid reamer has the propensity to push adjacent bone fragments apart instead of cutting a hole of the desired size. This can impact location, rotation, and alignment (e.g., reduction) of the bone fragments.

Turning now to FIGS. 20A-21B, a coring reamer instrument 200 is shown according to one embodiment. The coring reamer 200 is configured to create a hole or opening in the axial end of a long bone to access the medullary canal while minimizing the likelihood of separating the fracture or bone fragments. The coring reamer 200 is configured to cut and collect material within the body of the reamer 200, which may be later removed and used as a bone graft if desired. The coring reamer 200 extends from a proximal end 202 configured to interface with a handle (e.g., a quick connect handle) to a distal end 204 configured to cut bone along a central longitudinal tool axis. The coring reamer 200 includes a coring tip 206 configured to cut and collect bone, a core ejector 208 configured to push out the collected bone, and a reamer shaft 210 configured to support and move the core ejector 208.

The coring tip 206 includes a hollow cylindrical body or tubular shaft 212 with a through opening 214 configured to receive cut bone. The coring tip 206 includes front-cutting teeth 216 at the diameter of rotating shaft 212 with a hollow core, which avoids potential splitting of a reduced fracture by not pushing material outward, but instead packing material to be removed inside the device. The teeth 216 may by spaced and aligned around the circumference of the distal end of the coring tip 206. The teeth profiles may be straight, angled, or other geometrical shapes configured to enhance cutting efficiency and direct the cut material inside the coring tip 206.

The hollow opening 214 may be cylindrical such that as the cut bone packs the opening 214 it forms a cylindrical block or core of packed bone. One or more windows on the sides of the coring tip 206 may be in fluid communication with opening 214 to visualize the amount of bone collected and/or to facilitate removal of the bone. Advancement of instrument 200 cuts out the cylindrical block of material that ends up packed in the hollow core of the reamer 200, which may be removed as one single piece.

This packed bone material may be pushed out of the core using a sliding ejector component 208 and used as an autologous bone graft where necessary. The core ejector 208 may be a hollow tube with a proximal portion 218 to be gripped by the user and a distal portion 220 receivable inside the coring tip 206. An external surface of the proximal portion 218 of the ejector 208 may be patterned with a series of ridges or grooves or other textures to enhance grip such that the user is able to slide the ejector 208 along the longitudinal tool axis. The distal portion 220 of the ejector 208 may have a cylindrical body sized and dimensioned to be received inside the hollow openings 214 through the coring tip 206. As the ejector is advanced proximally, the ejector 208 pushes the bone core out of the coring tip 206.

The reamer shaft 210 is configured to support the core ejector 208 and to attach to a user handle. The reamer shaft 210 may have a cylindrical body with a proximal member 222 and a distal member 224. The distal member 224 may have a cylindrical body sized and dimensioned to be received through the core ejector 208. The proximal end 202 of the reamer shaft 210 may include a connector interface 226, such as a male quick-connect, for attaching to a quick-connect handle. The male connector 226 may be configured to connect to a power tool (e.g., power drill) to rotate the assembly for cutting with coring tip 206.

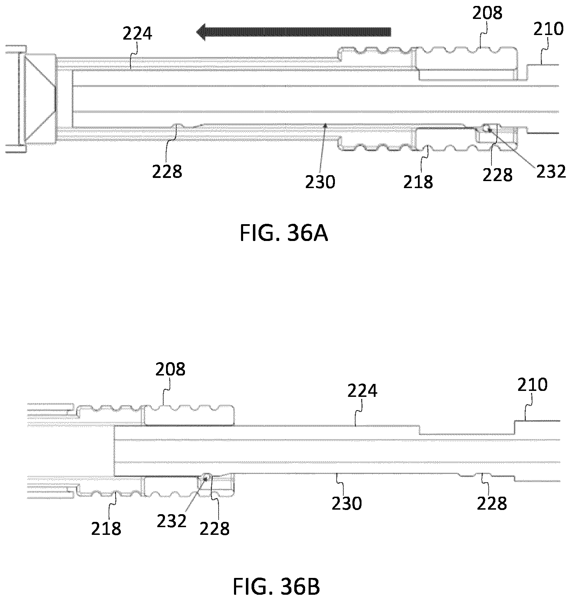

With further emphasis on FIGS. 36A-36B, the location or relative position of the reamer shaft 210 relative to the core ejector 208 may be locked. The distal member 224 of the reamer shaft 210 extends through the core ejector 208, and the inner surface of the core ejector 208 may engage with the external surface of the reamer shaft 210 at discrete locations. In one embodiment, the distal member 224 of the reamer shaft 210 includes locking grooves 228 and a wire track 230 configured to receive and guide a spring wire 232 attached to core ejector 208. FIG. 36A shows the core ejector 208 with the proximal grip 218 retracted on the reamer shaft 210 such that the wire 232 is received in locking groove 228. As the core ejector 208 moves distally to expel the bone core, the spring wire 232 on ejector 208 interfaces with the wire track 230. As shown in FIG. 36B, at its distal-most point, the wire 232 is received in a second locking groove 228 on the reamer shaft 224. As the core ejector 208 slides along the reamer shaft 210, the engagement of wire 232 on the ejector 208 may provide tactile and locked locations along the shaft of the reamer 210. Although a wire/groove engagement interface is exemplified, it will be appreciated that other suitable locking mechanisms may also be used.

The coring reamer 200 may be used to cut a hole of a precise diameter into the axial end of the long bone to access the medullary canal for the intramedullary nail. The coring reamer 200 is able to cut bone and collect the cut bone within the body of the reamer 200. The cut bone may be packed into a core, which may be easily removed with the core ejector 208. The coring reamer 200 may help to minimize the likelihood of separating the bone fragments or worsening the fracture during cutting.

### Trochanteric Nail Guide Wire Pusher

Trochanteric nails may be positioned within the femoral canal via the greater trochanter to secure multiple types of fractures in the upper part of the femur. During preparation for the insertion of the nail, the medullary canal may be reamed. To begin reaming, the surgeon may first insert a ball-tip guide wire through the full length of the medullary canal. A cannulated reamer head may be connected to a cannulated reamer shaft, and the reamer shaft may be connected to a cannulated power drill for reaming. The cannulated reaming instruments may be passed over the ball-tip wire to guide the reamer through the length of the canal. When reaming is completed, the reaming instruments are removed, however, the ball-tip guidewire may remain in the canal to guide nail insertion. A common challenge during this procedure step is that the ball-tip wire can come out with the reaming instruments when they are removed. This challenge may be mitigated with the use of a guidewire pusher.

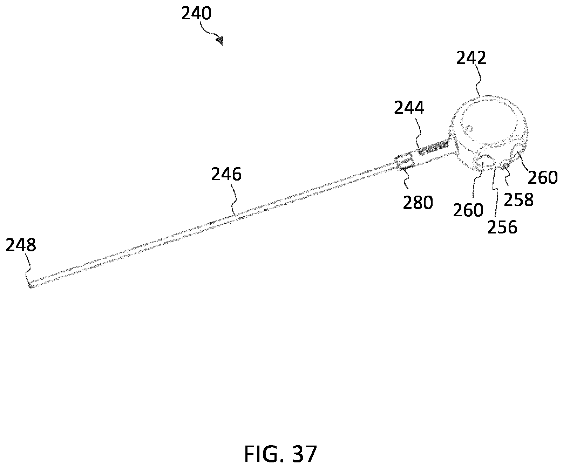

Turning now to FIGS. 37-43, a guidewire pusher 240 is shown according to one embodiment. The guidewire pusher 240 may be used to keep the ball-tip guidewire in place as the reamer and reamer shaft are removed from the medullary canal. As shown in FIG. 37, the guidewire pusher 240 may include a proximal knob 242, a neck 244, and a long shaft 246 with a distal tip 248 for pushing the ball-tip guidewire 250. The knob 242 may have a rounded outer shape, such as a cylinder with flattened or contoured side surfaces, which is easily held by the user. The guidewire pusher 240 may include one or more ball-tip wire engagement surfaces for interacting with the ball-tip guidewire 250.

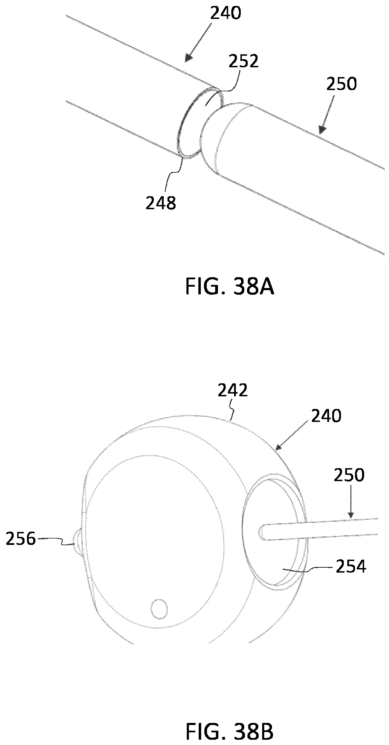

FIGS. 38A-38B show examples of ball-tip wire engagement with the guidewire pusher 240. As the name implies, the ball-tip wire 250 is a wire with ends having a spherical shape minimizing sharp edges and points. In FIG. 38A, the distal tip 248 of shaft 246 defines a concave recess 252 sized and dimensioned to receive the ball-tip of wire 250. In this embodiment, the shaft 246 of guidewire pusher 240 may have a diameter equal to the ball-tip wire 250, which can be used to push the wire 250 sub-flush in a cannula. In FIG. 38B, the guidewire pusher 240 defines an enlarged recess 254 defined into one side of the proximal knob 242. The recess 254 may have a circular shape with a diameter larger than wire 250. The recess 254 may include a flat or a concaved bottom surface for contacting the end of wire 250. In this embodiment, the larger wire engagement recess 254 may be used to hold wire 250 in place as the reaming instruments are removed.

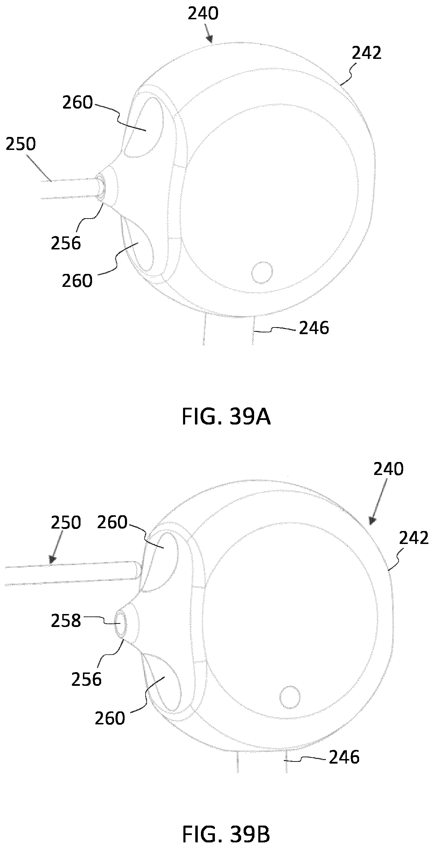

FIGS. 39A-39B show three separate fine engagement surfaces that can be used to engage the ball-tip wire 250. In FIG. 39A, the proximal knob 242 has a protruding mouth 256 with a cupped or concave recess 258 for holding the end of the ball-tip wire 250. The mouth 256 may be positioned across the knob 242 opposite from enlarged recess 254. In FIG. 39B, indentations 260 may be positioned above and below mouth 256. The indentations 260 may be angled or sloped with flat or rounded bottom surfaces configured to contact the end of wire 250, thereby providing precise engagement with the ball-tip wire 250. The guidewire pusher 240 includes several options for engaging with the ball-tip guidewire 250 when working through cannulated instruments. This variety of alternatives combine to create an instrument that is adaptable to various applications and surgeon preferences.

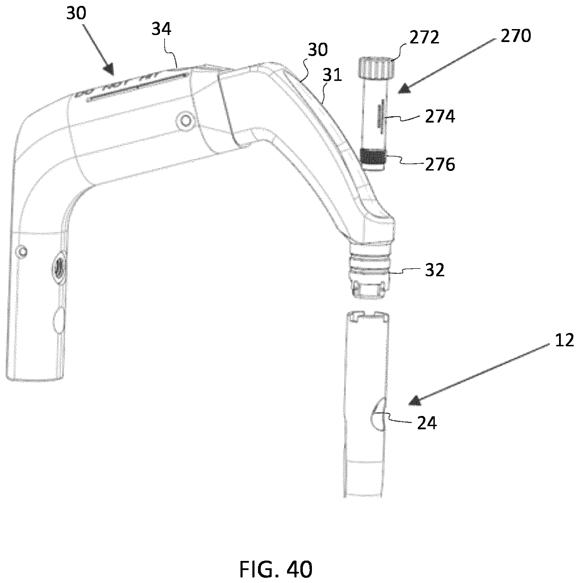

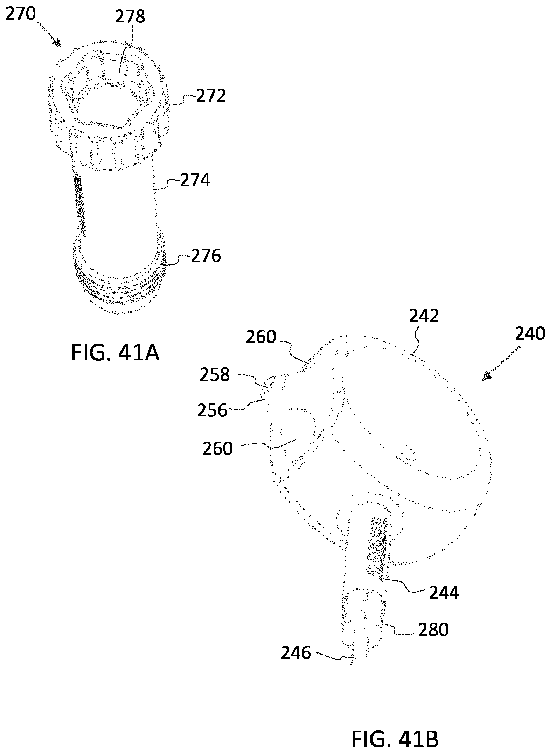

Turning now to FIGS. 40-43, in addition to the guidewire engagement function, the guidewire pusher 240 may be used to provisionally assemble the trochanteric nail 12 to the nail insertion instrument, such as nail implant holder 31. In this embodiment, the trochanteric nail 12 may be secured to the nail implant holder 31 with coupling portion 32 and a cannulated nail connection bolt 270. As shown in FIG. 41A, the cannulated nail connection bolt 270 may include top or head 272, cylindrical shank 274, and threaded portion 276 configured to engage with a corresponding thread in the nail 12.

The head 272 of connection bolt 270 may have an outer surface with protrusions or teeth extending around the external circumference of the head 272. An upper surface of the head 272 may define a drive recess 278 configured to receive a driver to assemble and tighten the implant 12 to the insertion handle 30. The drive recess 278 is also configured to mate with a corresponding key 280 at the base of neck 244 on the guidewire pusher 240. In one embodiment, the nail connection bolt 270 defines a hex drive recess 278, which is configured to be engaged by a hex driver instrument (not shown) and a hex key 288 of the guidewire pusher 240. Although a hex mating engagement is exemplified, it will be appreciated that other suitably shaped recesses and keys or other engagement is contemplated.

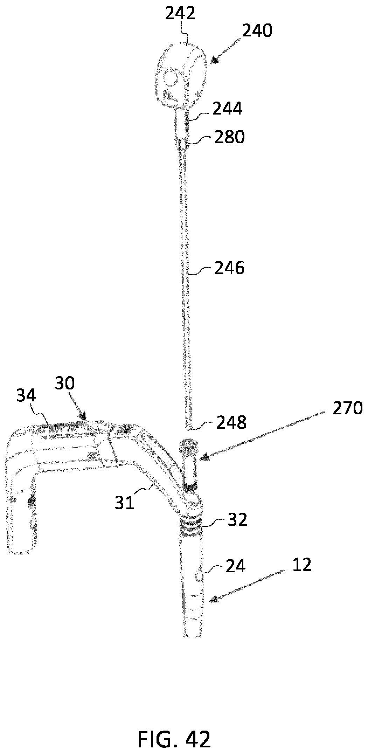

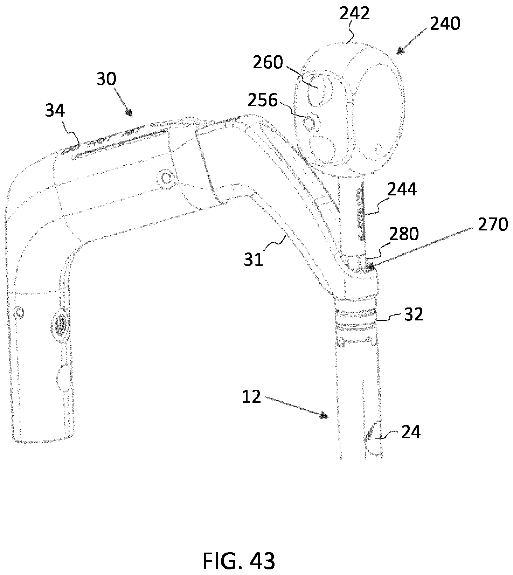

The mating drive interface 280 of the guidewire pusher 240 may help to aid in the assembly of the construct. As shown in FIG. 42, the distal tip 248 of the guidewire pusher 240 may be inserted through the nail connection bolt 250, the nail implant holder 31, and through the intramedullary nail 12. As shown in FIG. 43, the long shaft 246 of the guidewire pusher 240 may be used to axially orient these three components. The key 280 of the guidewire pusher 240 may be used to engage and advance the connection bolt 270 through the nail implant holder 31 and into the nail 12. In this manner, the guidewire pusher 240 may be used to aid in the assembly of the trochanteric nail 12 with the insertion handle 30 and connection bolt 270. It can often be difficult for a single person to assemble these three instruments at once using a standard hex driver. The guidewire pusher's ability to capture and orient all components turns a cumbersome task into an efficient two hand operation.

### Intramedullary Nail Distal Targeting System

Intramedullary nails may be positioned within the medullary canal of a long bone to treat fractures. Trochanteric nails may be inserted into the canal of the femur through the greater trochanter. Antegrade nails may involve entry through either the piriformis fossa (PF) or greater trochanteric (GT) tip of the femur. In addition to proximal fixation (e.g., lag screw), bone anchors or fasteners may also be inserted through the distal end of the nail to provide distal locking. A targeting system may be useful to help accurately and reliably insert the distal fasteners into the intramedullary nail.

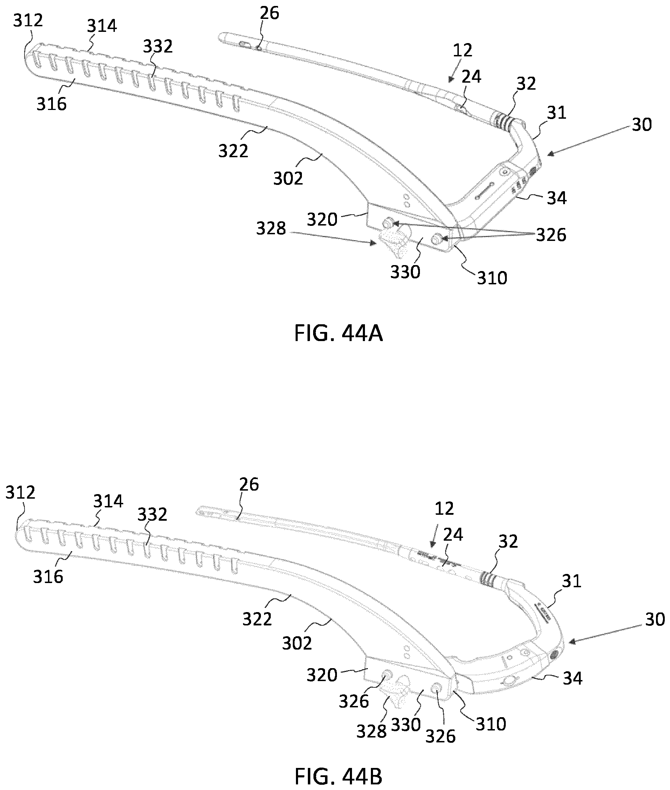

Turning now to FIGS. 44A-51, a distal targeting system 300 is shown according to one embodiment. The distal targeting system 300 is configured to target screw placement in the distal slots and holes 26 of several types of intramedullary nails 12, including trochanteric long nails, antegrade greater trochanteric nails, and antegrade piriformis fossa nails. For example, FIG. 44A shows one embodiment of a trochanteric long nail 12 with a troch insertion handle 30 and FIG. 44B shows one embodiment of an antegrade nail 12 with an antegrade insertion handle 30.

Regardless of the type of nail and insertion handle, the distal targeting system 300 provides surgeons with a way to aim and insert fasteners and screws accurately and reliably through the distal holes 26 of the intramedullary nail 12. The distal targeting system 300 enables the surgeon to target both left and right nails 12, for example, ranging in length at suitable increments. The interchangeable nail compatibility offers a wide range of functionality for surgeons with a single instrument set. The distal targeting system 300 also allows for deflection adjustment to account for deflection in the nail 12 that may occur upon entry into the medullary canal.

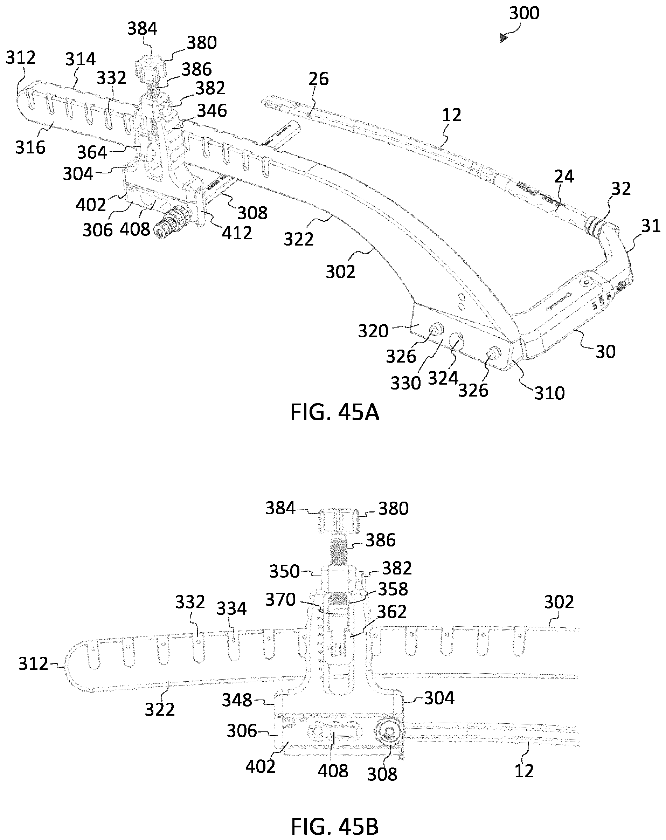

As shown in FIG. 45A, the distal targeting system 300 includes a distal aiming arm 302 attachable to nail insertion handle 30 which is coupled to intramedullary nail 12, a deflection assembly 304 attachable to the distal aiming arm 302, a targeting module 306 attachable to the deflection assembly 304, and a sleeve 306 positionable through the targeting module 306, which sets the trajectory of the distal fasteners to interface with the distal openings 26 of the nail 12. The distal aiming arm 302 has a body extending from a proximal end 310, which attaches to the nail insertion handle 30, to a distal end 312, which is a free end. An inner face 314 points toward the intramedullary nail 12 and an opposite outer face 316 points outward.

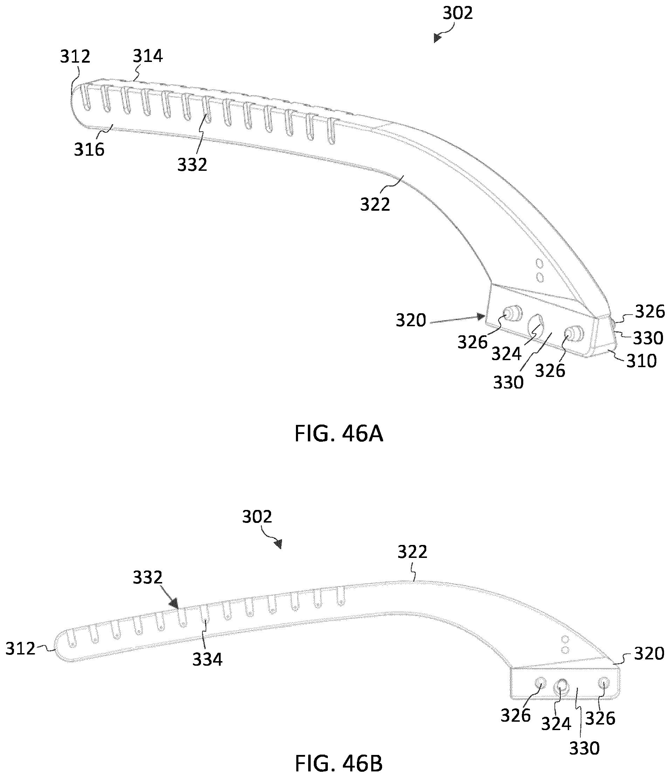

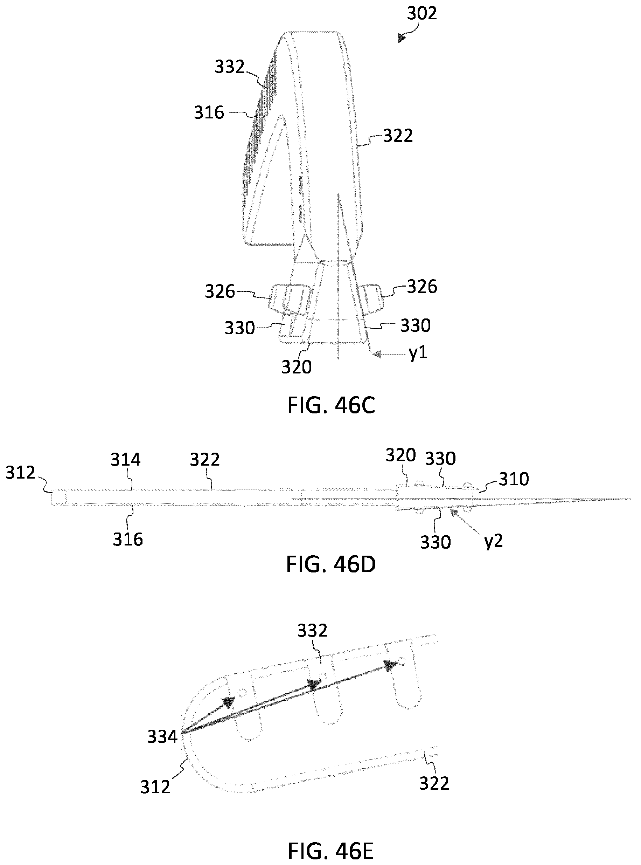

With further emphasis on FIGS. 46A-46B, the distal aiming arm 302 includes an attachment head 320, which connects to the insertion handle 30, and a plate 322 for supporting the deflection assembly 304. The attachment head 302 is designed to connect with any nail insertion handle 30, such as a troch nail insertion handle or an antegrade insertion handle. The attachment head 320 may be an angled block with one or more through holes 324 configured to align with mating holes in the insertion handle 30. For example, two outer holes 324 may be configured to receive alignment pins 326 and a middle hole 324 may be configured to receive a connection screw 328. In order to assemble the distal aiming arm 302 to the insertion handle 30, the two alignment pins 326 on the attachment head 320 are assembled with the mating holes in the insertion handle 30 and then the connection screw 328 is used to tighten the assembly and facilitate rigid targeting.

With further emphasis on FIGS. 46C-46D, the attachment head 320 may include two mirrored connection surfaces 330 to facilitate the targeting of left and right nails 12. The connection surfaces 330 may have two angles y1, y2 that act in different planes. A first angle y1 of the connection surface 330 may be present to account for the anteversion of the nail 12 (e.g., 12° angle accounts for 12° anteversion of nail). In one embodiment, the distal locking holes 24 may be rotated around the longitudinal axis of the nail 12 by 12° relative to the proximal holes 22 to account for the anatomy of the femur. The second angle y2 of the connection surface 330 may be present to account for the lateral bend of the nail 12 (e.g., 2.5° angle for 5° lateral bend of the nail). For example, the trochanteric long nail and antegrade greater trochanteric nail may have the 5° lateral bend while the antegrade piriformis fossa nail may lack a lateral bend.

The plate 322 of the distal aiming arm 302 may extend distally from the attachment head 320. The plate 322 may be substantially flat between the inner and outer faces 314, 316. The plate 322 may have an arcuate bend or curve from the attachment head 320 and a generally straightened section toward the distal end 312. The inner and outer faces 314, 316 of the plate 322 may define a plurality of attachment recesses or dovetails 332 configured to secure the deflection assembly 304. As best seen in FIG. 45B, the attachment dovetails 332 are designed to accommodate incremental placement of the deflection assembly 304. The dovetails 332 may be present on both sides 314, 316 of the aiming arm 302 to enable reversibility between left and right nails 12. The dovetails 332 may include a series of indentations or female recessed areas along one side of each face 314, 316 configured to receive a foot 366 of the deflection assembly 304. In one embodiment, the dovetails 332 may be equally spaced apart at a defined increment (e.g., 20 mm increments). The dovetails 332 may be positioned to target nail lengths, for example, ranging from 240mm to 480mm at 20mm increments. Each female dovetail 332 may define an arched surface at the bottom of the dovetail 322 configured to receive a corresponding arched surface on the foot 366 of the deflection assembly 304.

Each dovetail 332 may contain a ball detent divot 334 configured to receive a ball detent 368 of the deflection assembly 304. The ball detent divot 334 is a partially spherical recess sized and dimensioned to receive a portion of the ball detent 368. The ball detent divots 334 may be located within each dovetail 322 at any suitable location to align with the deflection assembly 304. After the deflection assembly 304 is connected to the aiming arm 302 via dovetail 332, the ball detent 368 on the deflection assembly 304 clicks into the ball detent divot 334. This retains the deflection assembly 304 in place and provide tactile feedback to the user that the part is fully seated.

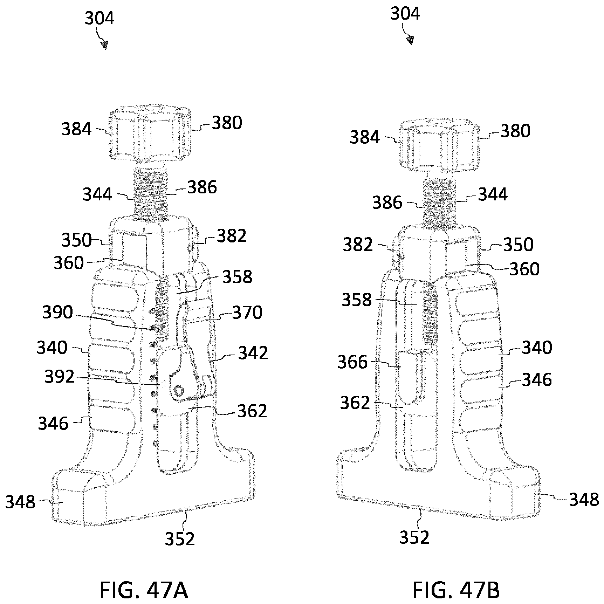

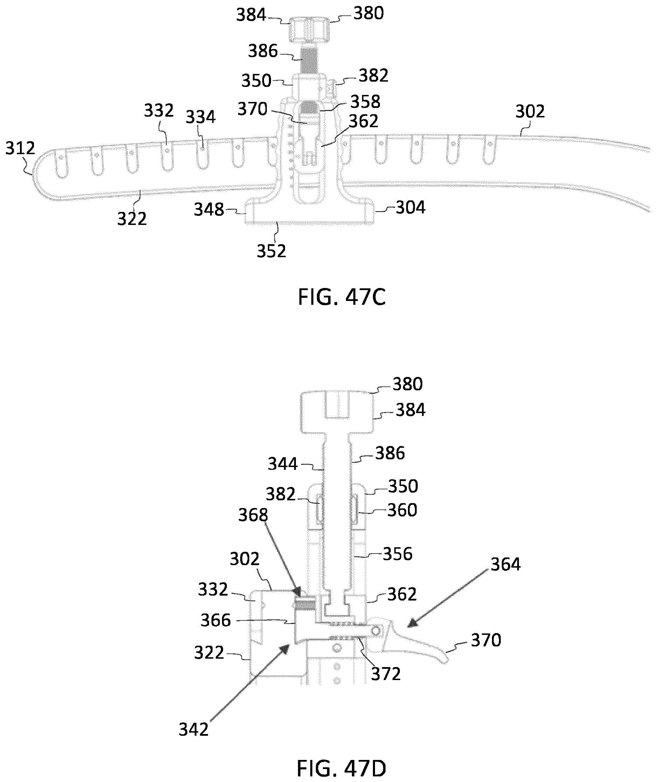

FIGS. 47A-47G show deflection assembly 304 according to one embodiment. The deflection assembly 304 allows for vertical adjustment of the targeting trajectory to account for anterior or posterior deflection as the intramedullary nail 12 is inserted into the medullary canal. The deflection assembly 304 may include an outer casing 340, a dovetail assembly 342 for attachment to the distal aiming arm 302, and an adjustment assembly 344 for adjusting vertical position of the deflection assembly 304 relative to the distal aiming arm 302.

The outer casing 340 may include a handle portion 346 for retaining the deflection assembly 304, a base 348 for securing the targeting module 306, and a neck 350 for retaining the vertical adjustment assembly 344. The handle portion 346 may have grips for holding the deflection assembly 304 by a user. The base 348 may have an enlarged width with a bottom surface 352 configured to mate with the targeting module 306. For example, the bottom surface 352 of the casing 340 may define a dovetail groove 354 configured to receive a corresponding tenon 410 on the targeting module 306. A blind hole 356 may extend vertically through the neck 350 and handle portion 346 of the casing 340. Opposite vertical windows 358 are provided through the handle portion 346 of the casing 340 and in fluid communication with the blind hole 356 to provide for vertical movement of the dovetail assembly 342. A transverse window 360 is provided through the neck 350 of the casing and in fluid communication with the blind hole 356 to receive the coarse adjustment thread release.

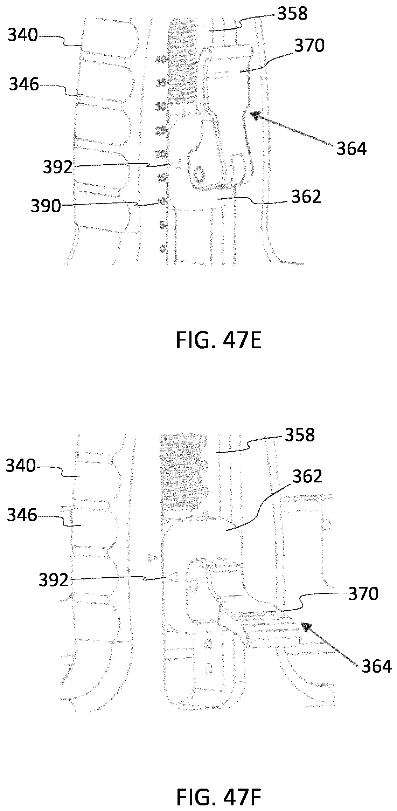

The deflection assembly 304 is attached to the distal aiming arm 302 with dovetail assembly 342. The dovetail assembly 342 includes a moveable block 362 and a cam lock 364 attached to the male dovetail or foot 366 having a ball detent 370. As best seen in FIG. 47B, the foot 366 includes a male dovetail projection with a planar outer surface and an arched bottom surface configured to be received into the corresponding female dovetail recess 332 in the plate 322. Once the deflection assembly 304 is attached to the distal aiming arm 302, the cam lock 364 is used to secure the attachment. The cam lock 364 functions using a cam lever 370 which connects to the male dovetail or foot 366 in the aiming arm 302. The foot 366 is positioned on one side of the block 362 extending through window 358 and the cam lever 370 is positioned on the opposite side of the block 362 extending through the opposite window 358. The internal compression spring 372 maintains preload on the male dovetail 366 to ensure easy assembly with the aiming arm 302. FIG. 47E shows the cam lock 364 in a locked position where the cam lever 370 is pivoted upward to lock the dovetail assembly 342. FIG. 47F shows the cam lock 364 in an un-locked position where the cam lever 370 is pivoted outward.

The adjustment assembly 344 provides for vertical adjustment of the deflection assembly 304 relative to the distal aiming arm 302. The vertical adjustment of the deflection assembly 304 may be manipulated by two different methods: fine and course adjustments. The fine deflection adjustment allows the user to fine tune the vertical position of the deflection assembly 304. The coarse deflection adjustment enables the user to more quickly adjust the targeting system to account for nail deflection. The adjustment assembly 344 includes a fine adjustment knob 380 and a coarse adjustment button 382. The fine adjustment knob 380 includes an upper knob 384 attached to a threaded shaft 386, which translates the rotational motion of knob 384 to vertical movement of the block 362. The coarse adjustment button 382 is retained in the neck 350 of the casing 340 through window 360 and a portion of the button 382 protrudes through window 360. The threaded shaft 386 of the fine adjustment knob 380 extends through the neck 350, through the coarse adjustment button 382, and though the blind hole 356 in the body of the casing 340.

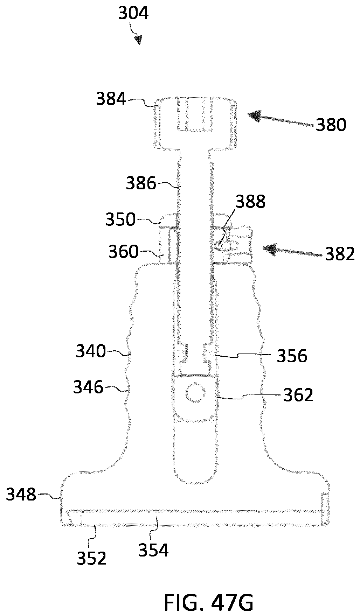

As best seen in FIG. 47G, the coarse adjustment button 382 defines a threaded through opening that engages/disengages from the threads of the threaded shaft 386. A pin 388 slides along a slot and prevents disassembly from the neck 350. In the default position, the threaded shaft 386 may be engaged by the coarse adjustment mechanism. The distal end of the shaft 386 is secured in the top of block 362. The threaded knob 380 enables the surgeon to make fine deflection adjustments. The thread release button 382 allows the surgeon to quickly make larger adjustments and enables course adjustment of the vertical position. When the thread release button 382 is pressed, the threads are disengaged from shaft 386, allowing free motion up and down. Ball detents may be placed along the full range of adjustment, for example, at 5mm increments to provide tactile feedback to the surgeon as the instrument is adjusted. The user may also use the threaded knob 380 for fine manipulation of the vertical adjustment. A single turn of knob 380 may result in 1mm of vertical travel, for example, allowing the user to make minor adjustments to the instrument position.

As best seen in FIG. 47E, the casing 340 of deflection assembly 304 may include one or more markings 390, such as graduated markings, along the edge of the window 358. The markings 390 may be etched or printed onto the outer face of the handle 346, for example, with points, lines, numbers, or other visual symbols to signify a vertical height or position of the deflection assembly 304. An indicator 392 may also be etched or printed into the face of the block 362. As the block 362 travels vertically along window 358, the indicator 392 on the block 362 shows the vertical reading to the user. The markings 390 may be used to quickly locate the desired positioning.

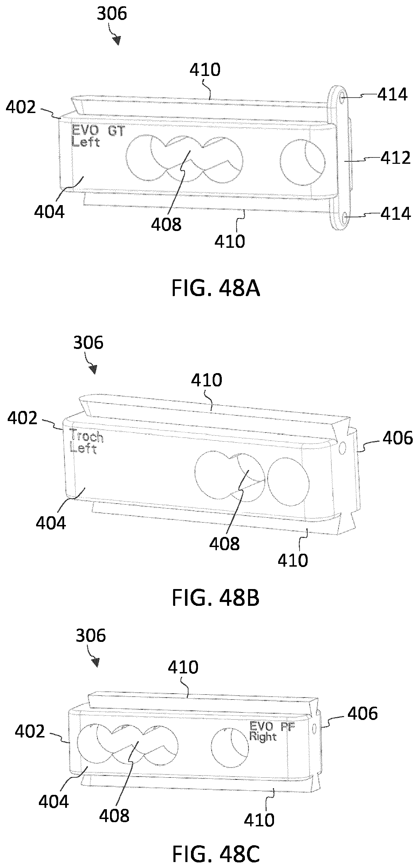

Turning now to FIGS. 48A-48C, examples of targeting modules 306 are shown. In order to precisely target the specific type of nail 12 (e.g., trochanteric nail, antegrade greater trochanter, antegrade piriformis fossa), different targeting modules 306 may be used. FIGS. 48A-48C show three different types of targeting modules 306: antegrade greater trochanter (GT) in FIG. 48A; trochanteric nail in FIG. 48B; and antegrade piriformis fossa (PF) in FIG. 48C. The targeting modules 306 are configured for targeting left and right nails 12. It will be appreciated that any suitable configuration may be used to target the specific intramedullary nail 12.

Each targeting module 306 may include a targeting block with an inner face 404 pointing toward the intramedullary nail 12 and an opposite outer face 406 pointing outward. It will be appreciated that the sides may be reversible depending on the handedness of the nail 12. One or more openings or holes 408 extend between the inner and outer faces 404, 406, which are positioned to accurately target the distal holes 26 in the nail 12, including the dynamic slot position and the static slot positions in each nail type. The holes 408 may be distinct or overlapping. The trajectory of these holes 408 may be placed at a +2.5° angle for the troch and GT modules. This angle adds to the 2.5° angle on the aiming arm attachment head 320 to align with the 5° lateral bend featured on the troch and GT nails. The PF module may contain a -2.5° angle to cancel the 2.5° angle on the aiming arm attachment head 320 to align with the PF nail, which contains no lateral bend.

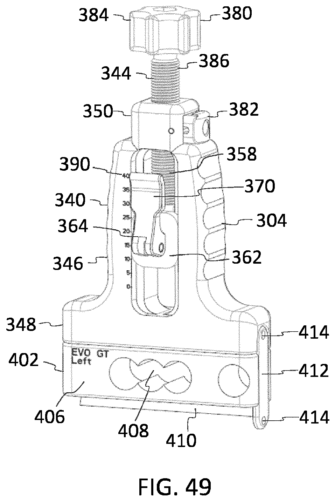

As shown in FIG. 49, the targeting module 306 is attached to the deflection assembly 304. In one embodiment, the targeting module 306 includes a tenon 410 configured to fit within the corresponding groove 354 in the bottom surface 352 of the casing 340 of the deflection assembly 304. Tenons 410 may be projecting from each of the upper and lower surfaces of the block 402 so that the block 402 may be reoriented and reversed depending on the nail. The joint between the targeting module 306 and the deflection assembly 304 may be a sliding dovetail interface between the tenon 410 and dovetail groove 354. In one embodiment, the targeting module 306 is attached to the deflection assembly with a snap fit design, which allows for quick attachment. The attachment may be further secured with an optional side bracket 412 extending along one side of the block 402. The side bracket 412 may be integral with the block 402 or a separate component. The side bracket 412 may include one or more openings 414 configured to receive fasteners, such as threaded screws, to further secure the side bracket 412 to the base 348 of the deflection assembly 304. The threaded interface may facilitate a more robust connection of the module 306 to the deflection assembly 304 for rigid targeting.

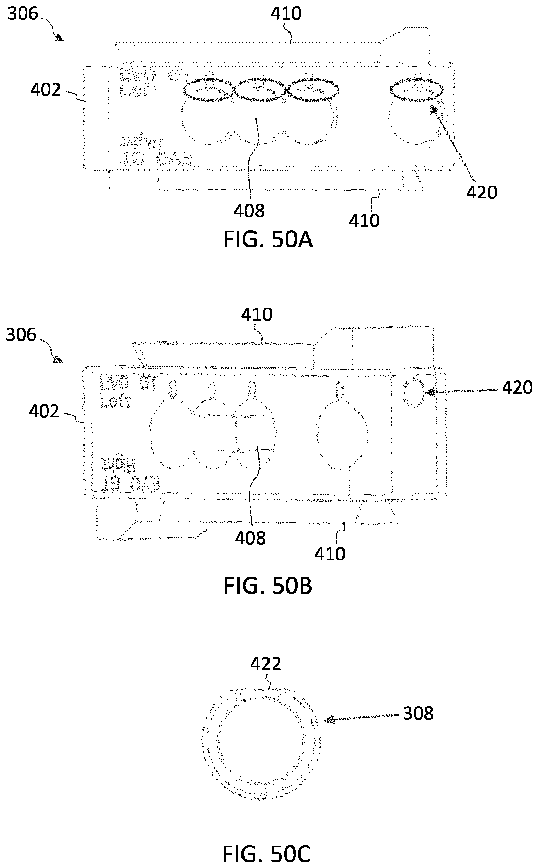

As shown in FIGS. 50A-50C, the targeting holes 408 in targeting module 306 may include one or more retention pins 420 configured to retain the sleeve 308 therein. Similar to retention pins 76, retention pins 420 are configured to secure a D-shaped sleeve (as shown in FIG. 50C). The module holes 306 may include PEEK retention pins 420 for self-retention of the driver sleeve 308. The PEEK retention pins 420 may be positioned to interfere with the targeting holes 408. For example, one retention pin 420 may be positioned along one side of each targeting hole 408 such that the pin 420 intersects the module hole 408. The sleeve 308 may have a D-shaped shaft which interfaces with the PEEK retention pin 420. The D-shaped shaft of sleeve 308 has a cross-sectional profile resembling a D shape where a portion of the shaft defines a flat side 422. In order to engage this self-retention feature, the sleeve 308 is inserted with the flat 422 of the shaft oriented parallel to the longitudinal axis of the PEEK pin 420 and then rotated 180° so the shaft interferes with the retention pin 420. The resulting friction fit retains the sleeve 308 within the targeting hole 408.

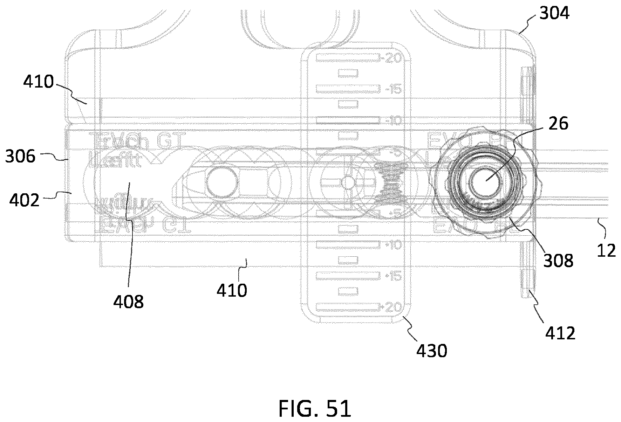

FIG. 51 shows sleeve 308 positioned through one opening 408 in the targeting module 306 to align with a distal opening 26 of the intramedullary nail 12. The system 300 may also include an alignment display 430 to provide the surgeon with quantitative information to determine exactly how much deflection has occurred. This enables the surgeon to adjust the targeting system more quickly and reliably to the optimal position. The alignment display 430 may be a radiolucent instrument with radiopaque measurement markings attachable to the targeting module 306 or integral with the targeting module 306. The measurement markings, such as graduated markings, may include points, lines, numbers, or other visual symbols to signify the vertical position of the targeting module 306. The alignment display 430 may include radiopaque markers that are visible under x-ray or other imaging, which inform the user with a precise measurement of the nail deflection. The alignment display 430 enables the surgeon to quantify the amount of adjustment needed and clearly identify when the system has reached the optimal targeting position at the center of the nail hole 26. This allows users to more quickly and accurately reach the optimal trajectory for screw placement intraoperatively.

As best seen in FIG. 45A, once the full targeting system 300 is assembled, sleeve 308 (e.g., a 5mm locking screw sleeve) is inserted into the module hole 408 that corresponds with the hole 26 or slot that the user intends to target in the nail 12. The distal targeting system 300 provides targeting holes 408 in which one or more sleeves 308, such as screw sleeves, drill sleeves, and/or driver sleeves, can be inserted. The course and fine adjustment 380, 382 may be used to adjust the sleeves 308 to the optimal trajectory, for example, under x-ray guidance or other imaging. The system 300 rigidly holds these sleeves 308 in the desired trajectory during drilling and screw insertion. The targeting system 300 provide surgeons with a way to insert locking screws accurately and reliably through the distal end of the intramedullary nail.

It will be further understood that various changes in the details, materials, and arrangements of the parts which have been described and illustrated in order to explain the nature of this invention may be made by those skilled in the art without departing from the scope of the invention as expressed in the claims. One skilled in the art will appreciate that the embodiments discussed above are non-limiting. It will also be appreciated that one or more features of one embodiment may be partially or fully incorporated into one or more other embodiments described herein.

## Claims

1. A targeting system (300) for inserting a lag screw (14) into a trochanteric intramedullary nail (12), the system (300) comprising:
a trochanteric intramedullary nail (12) having a proximal end (20) with a proximal through opening (24) and a distal end (22);
a nail insertion handle (30) including a coupling portion (32) to removably couple to the proximal end (20) of the intramedullary nail (12), a handle portion (34), and an aiming guide (36) having an opening (38) that aligns with the proximal through opening (24) in the intramedullary nail (12);
wherein the handle portion (34) includes an anteversion alignment system (40) configured to optimally insert a lag screw (14) into the proximal through opening (24) of the trochanteric intramedullary nail (12) and into the femoral neck, and a lag screw aiming assembly configured to reliably insert down to the bone, thereby defining a trajectory to the proximal through opening (24) in the intramedullary nail (12),
**characterised in that** the targeting system further comprises an anti-rotation system configured to stabilize the bone when the lag screw is inserted,
wherein the anti-rotation system (60) includes a plurality of anti-rotation holes (62), anti-rotation sleeves (64) positionable through the holes, and anti-rotation guide wires (66) positionable through the sleeves (64) and into bone,
wherein the anti-rotation holes (62) include an upper pair of holes (62A) converging distally at a first angle (x1) and a lower pair of hole (62B) converging distally at a second angle, wherein the second angle (x2) is greater than the first angle (x1).

2. The targeting system (300) of claim 1, wherein the handle portion (34) defines an anteversion guide wire hole (42) along a center plane of the nail insertion handle (30) and a pair of radiopaque goal posts (44) positioned on opposite sides of the guide wire hole (42).

3. The targeting system (300) of claim 2, wherein the radiopaque goal posts (44) includes two parallel rows of pins retained in the handle portion (34), and when the goal posts (44) are centered in parallel and a guide wire positioned through the anteversion guide wire hole (42) is centered with a neck and head of the bone, an optimal anteversion position is found for lag screw (14) insertion.

4. The targeting system (300) of claim 1, wherein the anti-rotation holes (62) each include a retention pin (76) configured to secure the anti-rotation sleeve (64) in the anti-rotation hole (62).

5. The targeting system (300) of any one of the preceding claims, wherein the lag screw aiming assembly includes a driver sleeve positionable through the opening in the aiming guide, a wire sleeve with distal cutting flutes positionable through the driver sleeve, and a trocar positionable through the wire sleeve.

## Patentansprüche

1. Zielsystem (300) zum Einsetzen einer Zugschraube (14) in einen trochantären Marknagel (12), wobei das System (300) aufweist:
- einen trochantären Marknagel (12), der ein proximales Ende (20) mit einer proximalen Durchgangsöffnung (24) und ein distales Ende (22) aufweist;
- einen Nageleinsetzgriff (30) mit einem Verbindungsabschnitt (32) zum entfernbaren Verbinden mit dem proximalen Ende (20) des intramedullären Nagels (12), einem Griffabschnitt (34) und einer Zielführung (36) mit einer Öffnung (38), die mit der proximalen Durchgangsöffnung (24) im intramedullären Nagel (12) ausgerichtet ist;
- wobei der Griffabschnitt (34) ein Anteversions-Ausrichtungssystem (40), das eingerichtet ist, um eine Zugschraube (14) optimal in die proximale Durchgangsöffnung (24) des trochantären Marknagels (12) und in den Schenkelhals einzusetzen, und eine Zugschrauben-Zielanordnung umfasst, die eingerichtet ist, um zuverlässig bis zum Knochen eingeführt zu werden, wodurch eine Bewegungsbahn zur proximalen Durchgangsöffnung (24) im Marknagel (12) gebildet wird,
- **dadurch gekennzeichnet, dass** das Zielsystem ferner ein Anti-Rotationssystem aufweist, das eingerichtet ist, um den Knochen beim Einsetzen der Zugschraube zu stabilisieren,
- wobei das Anti-Rotationssystem (60) eine Mehrzahl von Anti-Rotationslöchern (62), durch die Löcher positionierbare Anti-Rotationshülsen (64) und Anti-Rotations-Führungsdrähte (66) umfasst, die durch die Hülsen (64) hindurch und in den Knochen positionierbar sind, wobei die Anti-Rotations-Löcher (62) ein oberes Lochpaar (62A), das distal unter einem ersten Winkel (x1) konvergiert, und ein unteres Lochpaar (62B) umfassen, das distal unter einem zweiten Winkel konvergiert, wobei der zweite Winkel (x2) größer ist als der erste Winkel (x1).

2. Zielsystem (300) nach Anspruch 1, wobei der Griffabschnitt (34) ein Anteversions-Führungsdrahtloch (42) entlang einer Mittelebene des Nageleinsetzgriffs (30) und ein Paar röntgendichter Zielpfosten (44) bildet, die auf gegenüberliegenden Seiten des Führungsdrahtlochs (42) positioniert sind.

3. Zielsystem (300) nach Anspruch 2, wobei die röntgendichten Zielpfosten (44) zwei parallele Reihen von Stiften umfassen, die im Griffabschnitt (34) gehalten werden, und wenn die Zielpfosten (44) parallel zentriert sind und ein Führungsdraht, der durch das Anteversionsführungsdrahtloch (42) positioniert ist, mit einem Hals und einem Kopf des Knochens zentriert ist, eine optimale Anteversionsposition für das Einsetzen der Zugschraube (14) gefunden wird.

4. Zielsystem (300) nach Anspruch 1, wobei die Anti-Rotationslöcher (62) jeweils einen Halte-Stift (76) umfassen, der eingerichtet ist, um die Anti-Rotationshülse (64) im Anti-Rotationsloch (62) zu befestigen.

5. Zielsystem (300) nach einem der vorhergehenden Ansprüche, wobei die Zugschrauben-Zielanordnung eine durch die Öffnung in der Zielführung positionierbare Antriebshülse, eine Drahthülse mit distalen Schneidrillen, die durch die Antriebshülse positionierbar ist und einen durch die Drahthülse positionierbaren Trokar umfasst.

## Revendications

1. Système de ciblage (300) pour l'insertion d'une vis de traction (14) dans un clou intramédullaire trochantérien (12), le système (300) comprenant :
un clou intramédullaire trochantérien (12) ayant une extrémité proximale (20) avec une ouverture traversante proximale (24) et une extrémité distale (22) ;
une poignée d'insertion de clou (30) comprenant une partie de couplage (32) pour se coupler de manière amovible à l'extrémité proximale (20) du clou intramédullaire (12), une partie poignée (34), et un guide de visée (36) ayant une ouverture (38) qui s'aligne avec l'ouverture traversante proximale (24) dans le clou intramédullaire (12) ;
dans lequel la partie poignée (34) comprend un système d'alignement en antéversion (40) configuré pour insérer de manière optimale une vis de traction (14) dans l'ouverture traversante proximale (24) du clou intramédullaire trochantérien (12) et dans le col du fémur,
et un ensemble de visée de vis de traction configuré pour s'insérer de manière fiable jusqu'à l'os, définissant ainsi une trajectoire vers l'ouverture traversante proximale (24) dans le clou intramédullaire (12),
**caractérisé en ce que** le système de ciblage comprend en outre un système anti-rotation configuré pour stabiliser l'os lors de l'insertion de la vis de traction,
dans lequel le système anti-rotation (60) comprend plusieurs trous anti-rotation (62), des manchons anti-rotation (64) positionnables à travers les trous, et des fils-guides anti-rotation (66) positionnables à travers les manchons (64), et dans l'os,
dans lequel les trous anti-rotation (62) comprennent une paire supérieure de trous (62A) convergeant distalement à un premier angle (x1) et une paire inférieure de trous (62B) convergeant distalement à un second angle, dans lequel le second angle (x2) est plus grand que le premier angle (x1).

2. Système de ciblage (300) selon la revendication 1, dans lequel la partie poignée (34) définit un trou de fil-guide en antéversion (42) le long d'un plan central de la poignée d'insertion de clou (30) et une paire de repères de visée radio-opaques (44) positionnés sur les côtés opposés du trou de fil-guide (42).

3. Système de ciblage (300) selon la revendication 2, dans lequel les repères de visée radio-opaques (44) comprennent deux rangées parallèles de broches retenues dans la partie poignée (34), et lorsque les repères de visée (44) sont centrés en parallèle et qu'un fil-guide positionné à travers le trou de fil-guide en antéversion (42) est centré sur un col et une tête de l'os, une position en antéversion optimale est trouvée pour l'insertion de la vis de traction (14).

4. Système de ciblage (300) selon la revendication 1, dans lequel les trous anti-rotation (62) comprennent chacun une broche de rétention (76) configurée pour fixer le manchon anti-rotation (64) dans le trou anti-rotation (62).

5. Système de ciblage (300) selon l'une quelconque des revendications précédentes, dans lequel l'ensemble de visée de la vis de traction comprend un manchon d'entraînement positionnable à travers l'ouverture du guide de visée, un manchon pour fil avec des cannelures de coupe distales positionnables à travers le manchon d'entraînement, et un trocart positionnable à travers le manchon pour fil.
